# EUROPEAN PATENT SPECIFICATION

(11) **EP 1 592 443 B1**
(45) Date of publication and mention of the grant of the patent: **30.03.2011**
(21) Application number: 03815990.1
(22) Date of filing: 13.08.2003
(51) Int. Cl.: A61K 39/07

(54) **IMPROVED ANTHRAX VACCINES AND DELIVERY METHODS**
VERBESSERTE ANTHRAXIMPFSTOFFE UND ABGABEVERFAHREN
AMELIORATIONS APPORTEES A DES VACCINS CONTRE L'ANTHRAX ET METHODES D'ADMINISTRATION

(30) Priority: 13.02.2003 US 446995 P; 14.07.2003 US 486460 P
(43) Date of publication of application: 09.11.2005
(73) Proprietor: Becton, Dickinson and Company, Franklin Lakes, New Jersey 07417-1880 (US)
(72) Inventor: MIKSZTA, John, A., Durham, NC 27713 (US); SULLIVAN, Vincent, J., Cary, NC 27560 (US)
(74) Representative: von Kreisler Selting Werner
(86) International application number: PCT/US2003/025158
(87) International publication number: WO 2004/073735

(56) References cited:
- EP-A1- 0 711 571
- EP-A1- 1 086 719
- WO-A-01/89622
- WO-A2-02/100340
- WO-A2-03/002069
- WO-A2-03/043574
- GB-A- 1 302 671
- US-A1- 2002 198 509
- US-B1- 6 329 156
- US-B1- 6 348 450
- PITTMAN P R ET AL: "Anthrax vaccine: immunogenicity and safety of a dose-reduction, route-change comparison study in humans" VACCINE, BUTTERWORTH SCIENTIFIC. GUILDFORD, GB, vol. 20, no. 9-10, 31 January 2002 (2002-01-31), pages 1412-1420, XP004334121 ISSN: 0264-410X
- MCBRIDE B W ET AL: "Protective efficacy of a recombinant protective antigen against Bacillus anthracis challenge and assessment of immunological markers" VACCINE, BUTTERWORTH SCIENTIFIC. GUILDFORD, GB, vol. 16, no. 8, May 1998 (1998-05), pages 810-817, XP004118489 ISSN: 0264-410X
- IVINS B ET AL: "Experimental anthrax vaccines: efficacy of adjuvants combined with protective antigen against an aerosol Bacillus anthracis spore challenge in guinea pigs" VACCINE, vol. 13, no. 18, 1995, pages 1779-1784, XP004057384
- ALEKSANDROV NI ET AL: "Aerosol immunization with dried live vaccines and toxoids. VI A study of postvaccination reactions and immunological efficacy of aerosol immunization with aerosol immunization with aerosol brucellosis, tularaemia, anthrax and plague vaccines in man" ZH MIKROBIOL EPIDEMIOL IMMUNOBIOL, vol. 32, 1961, pages 1245-1252, XP009076805

## Description

### Field of the Invention

The invention relates to immunogenic anthrax compositions, such as anthrax vaccines, and/or to the use of such compositions for preparing an anthrax vaccine.

### Background Information

In light of the events of September 11, 2001 and the anthrax scare in the following months, it has become increasingly clear that there is an urgent need for new biodefense vaccines to protect both military and civilian populations. Such new vaccine development may proceed in tandem with the development of novel delivery platforms in order to improve the efficacy of the candidate vaccines and to ensure safe and rapid deployment in the event of a national emergency.
The virulence of *Bacillus anthracis,* the causative agent of anthrax, is mediated by three main protein components: the protective antigen (PA), the lethal factor (LF) and the edema factor (EF). While these proteins are non-toxic individually, in combination they form a potentially lethal toxin. Cutaneous exposure to anthrax spores causes a skin lesion that typically responds well to antibiotic treatment. Inhalation anthrax, however, represents a much more formidable threat. Early diagnosis of inhalation anthrax is difficult and the disease often manifests abruptly as respiratory failure or hemodynamic collapse 2-4 days after the initial onset of symptoms.
The currently licensed vaccine in the United States is the Anthrax Vaccine Absorbed (AVA), which consists of a culture filtrate of a toxigenic, non-encapsulated strain of *B. anthracis,* absorbed onto aluminum hydroxide as adjuvant. While generally effective in providing protection against anthrax in humans, the current vaccine is administered in a 6-dose series over 18 months followed by yearly boosters thereafter. Thus, there is a need to produce safer and more potent anthrax vaccines, *e*.*g*., in order to reduce the number of required doses to achieve protective immunity.
A recently developed candidate anthrax vaccine is based on a recombinantly produced PA (Ivins, B.E., Infection and Immunity, 60:662-668, 1992; Ramirez, D.M., et al., J. Industrial Microbiol. and Biotechnol. 28:232-238, 2002). This vaccine is being investigated by USAMRIID as an alternative to AVA due to the potential for improved efficacy and thus a reduction in the required number of booster administrations, as well as an improved safety profile. Additional pre-clinical and clinical studies are required in order to determine the most effective delivery routes for protection and to further elucidate the potential requirements for adjuvants. Adjuvants such as alum are often associated with skin reactions and other adverse events; thus, a preferred vaccine would retain the immunogenicity of the alum-containing vaccine but would not have the associated side effects.

### Summary of the Invention

The present invention provides immunogenic anthrax compositions, such as anthrax vaccines, and the use of such compositions for preparing an anthrax vaccine.
One embodiment of the invention is an immunogenic anthrax composition (*e*.*g*., an anthrax vaccine) comprising dried solid particles having a volume mean diameter of between 35 µm and 300 µm, wherein at least 50% of said dried particles have a volume diameter within 80% of the mean, and said dried particles have a mean aerodynamic diameter of between 8 µm and 140 µm. Such a composition can be made by methods described herein and/or exhibits properties described herein.
Other embodiments of the invention include the use of the above compositions for preparing an anthrax vaccine. Such vaccine is suitable to be administered intranasally, intradermally (e.g. using hollow microneedles) and by topical-abrasion (e.g. using solid microneedles).
Compositions and vaccines of the invention exhibit several advantages. For example, they improve the immunogenicity and protective efficacy of vaccines; may reduce or eliminate the need for adjuvants in vaccine formulations; provide for minimally-invasive administration of vaccines; and/or enable self-administration of vaccines. In a preferred embodiment, the vaccine is a biodefense vaccine, more preferably an anthrax vaccine, and most preferably an anthrax rPA vaccine.

### Brief Description of the Drawings

Figure 1A is an elevated view of the handle end of a preferred embodiment of a microabrader.
Figure 1B is a side view of a preferred embodiment of a microabrader.
Figure 2A is a transparent perspective view of the microabrader device of Figures 1A and 1B.
Figure 2B is a cross sectional view of the microabrader device of Figure 1B.
Figure 3 is a side view of the abrading surface the microabrader device of Figures 1A, 1B, 2A, and 2B on the skin of a subject.
Figure 4 is a perspective view of the abrading surface in the embodiment of Figure 3. Figure 4A is a cross sectional side view of the abrader surface.
Figure 5 is a bottom view of the abrader surface of the embodiment of Figure 3.
Figures 6A-6F show the d56 serum concentrations of rPA-specific antibodies of the various isotypes in the experimental groups presented in Example 1.
Figures 7A-7C show the percent viability of monocyte cell cultures after incubation with lytic concentrations of PA and lethal toxin (LT) from *B. anthracis* and diluted serum from mice in different vaccination groups.
Figure 8 shows the percent viability of monocyte cell cultures after incubation with lytic concentrations of PA and lethal toxin (LT) from *B. anthracis* and diluted serum from rabbits in different vaccination groups.

### Detailed Description of the Invention

According to the present invention, the terms "improve", "improved" and "improvements" in reference to a vaccine are intended to mean that the vaccine stimulates a stronger immune response (*i*.*e*., provides increased immunogenicity) than that achieved via conventional formulations and/or via conventional routes of administration of the vaccine. Conventional formulations include, *e*.*g*., rPA in alum; and conventional routes of administration include, *e*.*g*., intramuscular [IM] or subcutaneous [SC]) delivery. The terms "improve", "improved" and "improvements" in this context can also mean that the vaccine stimulates a stronger protective immune response than that achieved via conventional formulations administered via conventional routes of delivery; and/or that the vaccine requires a reduced number of immunizations to achieve an immune response than that required for the conventional formulations administered via conventional routes; and/or that the vaccine requires a reduced number of immunizations to achieve a protective immune response than that required for conventional formulations administered via conventional routes.

In one aspect, the invention includes an immunogenic anthrax composition (*e*.*g*., an anthrax vaccine, or a pharmaceutical composition) in particulate form, which is suitable for delivery to a patient intranasally, and to methods for making and using such a composition. For example, one embodiment is an immunogenic anthrax composition in the form of dried particles made by a method comprising one or more of the following steps:
(i) atomizing a liquid formulation of an immunogenic anthrax composition to produce an atomized formulation,
(ii) freezing said atomized formulation to form solid particles, and
(iii) drying said solid particles to produce dried particles (*e*.*g*., a powder).

In preferred embodiments of this aspect of the invention, the immunogenic anthrax composition is an anthrax vaccine, preferably an Anthrax Vaccine Absorbed (AVA) vaccine, more preferably a recombinant protective antigen (rPA) vaccine. The immunogenic anthrax composition may or may not comprise an adjuvant.

The dried particles have a volume mean diameter of between 35µm and 300µm, wherein at least 50% of the dried particles have a volume diameter within 80% of the mean, and wherein said dried particles have a mean aerodynamic diameter of between 8µm and 140µm.

In a preferred embodiment, the dried particles of the immunogenic anthrax composition have a volume mean diameter of between 50µm and 300µm, more preferably between 50µm and 100µm, and/or the dried particles have a mean aerodynamic diameter of between 20µm and 70 µm.

With regard to this method of making the immunogenic anthrax composition (*e*.*g*., anthrax vaccine), the freezing may be performed by introducing the atomized formulation into a fluid or medium having a temperature below the freezing point of the liquid formulation; wherein preferably the fluid or medium has a boiling point or sublimation point lower than that of said atomized formulation. The fluid may be a gas or a liquid. The particles may be dried by lyophilization; or at about atmospheric pressure, in the presence of vibration, internals, mechanical stirring, or a combination thereof and are, optionally, further dried by lyophilization. The liquid formulation may comprise a mucoadhesive, *e*.*g*., chitosan, dermatan sulfate, chondroitin, or pectin. In one embodiment, the liquid formulation of the anthrax composition consists essentially of said anthrax composition and water.

The invention also includes an immunogenic anthrax composition (*e*.*g*., an anthrax vaccine) prepared by atomizing a liquid formulation of an immunogenic anthrax composition to produce an atomized formulation, such that, following freezing of said atomized formulation to form solid particles, and drying of said solid particles to produce dried particles, said dried particles have an average mean diameter of between 35µm and 300µm, at least 50% of said dried particles have a volume diameter within 80% of the mean, and said dried particles have a mean aerodynamic diameter of between 8µm and 140µm.

The invention also includes an immunogenic anthrax composition (*e*.*g*., an anthrax vaccine) that comprises dried particles having a mean aerodynamic diameter of between 8µm and 140µm and a volume mean diameter of between 35µm and 300µm, wherein at least 50% of said dried particles have a volume diameter within 80% of the mean, and a pharmaceutically acceptable carrier. This immunogenic anthrax composition may be an anthrax vaccine, *e.g*., an AVA vaccine or, preferably, an rPA vaccine.

Furthermore, the invention includes an immunogenic anthrax composition (*e*.*g*., an anthrax vaccine) made by a method comprising
(i) atomizing a liquid formulation of an immunogenic anthrax composition to produce an atomized formulation which comprises droplets having an average mean diameter of between 35µm and 300µm,
(ii) freezing said atomized formulation to form solid particles, and
(iii) drying said solid particles to produce dried particles.

In one preferred embodiment, the dried particles have a volume mean diameter of between 35µm and 300µm. Preferably at least 50% of the dried particles have a volume diameter within 80% of the mean, and said dried particles have a mean aerodynamic diameter of between 8µm and 140µm.

In another aspect, the invention provides a method of preparing an immunogenic anthrax composition (*e.g*., an anthrax vaccine). One preferred embodiment of this aspect of the invention is a method of preparing an immunogenic anthrax composition comprising
(i) atomizing a liquid formulation of an immunogenic anthrax composition to produce an atomized formulation,
(ii) freezing said atomized formulation to form solid particles, and
(iii) drying said solid particles to produce dried particles.

The dried particles have an average mean diameter of between 35µm and 300µm, at least 50% of said dried particles have a volume diameter within 80% of the mean, and the dried particles have a mean aerodynamic diameter of between 8µm and 140µm. In preferred embodiments, said dried particles have a volume mean diameter of between 50µm and 300µm, preferably between 50µm and 100µm; and/or said dried particles have an average mean aerodynamic diameter of between 20µm and 70µm. In particular embodiments of this method, freezing may be performed by introducing said atomized formulation into a fluid or medium having a temperature below the freezing point of said liquid formulation. Preferably, the fluid or medium has a boiling point or sublimation point lower than that of said atomized formulation. The fluid may be a gas or a liquid. The particles may be dried by lyophilization, or at about atmospheric pressure, in the presence of vibration, internals, mechanical stirring or a combination thereof. Drying may also be performed by a combination of drying at about atmospheric pressure, in the presence of vibration, internals, mechanical stirring or a combination thereof, and lyophilization. The liquid formulation may comprise a mucoadhesive, *e.g*., chitosan, dermatan sulfate, chondroitin, or pectin. In one particularly preferred embodiment, the liquid formulation of the immunogenic anthrax composition consists essentially of the immunogenic anthrax composition and water.

Another embodiment of this aspect of the invention is a method for preparing an immunogenic anthrax vaccine, comprising atomizing a liquid formulation of an immunogenic anthrax composition to produce an atomized formulation, such that, following freezing of said atomized formulation to form solid particles, and drying of said solid particles to produce a powder, the dried powder comprises dried particles having an average mean diameter of between 35µm and 300µm, at least 50% of said dried particles have a volume diameter within 80% of the mean, and said dried particles have a mean aerodynamic diameter of between 8µm and 140µm.

A further aspect of the invention is the use of the above compositions for preparing an anthrax vaccine Preferably, the anthrax vaccine, is an Anthrax Vaccine Absorbed (AVA) vaccine or a recombinant protective antigen (rPA) vaccine. The anthrax vaccine may additionally comprise an adjuvant, but is preferably adjuvant-free. The anthrax vaccine is suitable to be administered intranasally, and/or to a mucosal membrane.

With the above anthrax vaccine the amount of anthrax vaccine is reduced that is required to produce an efficacious result following intranasal administration to a patient in need thereof. With the anthrax vaccine an immune response can be elicited in a patient. The anthrax vaccine can be used in vaccinating a patient against anthrax. In one embodiment, the anthrax vaccine is administered as part of a prime-boost regimen. Suitable prime-boost regimens are familiar to those of skill in the art and can be determined by routine experimentation.

The above anthrax vaccine is suitable for intradermal (ID) delivery to a mammal, using a hollow microneedle (HMN). Accordingly, the anthrax vaccine, such as an Anthrax Vaccine Absorbed (AVA) vaccine, or, preferably, a recombinant protective antigen (rPA) vaccine is suitable for intradermal administration through at least one hollow needle having an outlet with an exposed height between 0 and I mm, said outlet being inserted into the skin to a depth of between 0.5 mm and 2 mm. The anthrax vaccine may or may not comprise an adjuvant.

In one embodiment of this anthrax vaccine is suitable to be administered with a device comprising
a hub portion being attachable to a prefillable reservoir storing the vaccine;
at least one hollow microneedle supported by said hub portion and having a forward tip extending away from said hub portion; and
a limiter portion surrounding said microneedle(s) and extending away from said hub portion toward said forward tip of said microneedle(s), said limiter including a generally flat skin engaging surface extending in a plane generally perpendicular to an axis of said microneedle(s) and adapted to be received against the skin of a mammal to administer an intradermal injection of the vaccine, said microneedle(s) forward tip(s) extending beyond said skin engaging surface a distance approximately 0.5 mm to 2.0 mm wherein said limiter portion limits penetration of the microneedle(s) into the dermal layer of skin of the mammal. In embodiments of this method, delivery of the immunogenic composition occurs at a depth between 0.025 mm and 2.5 mm in the skin of the mammal; the delivered immunogenic composition induces an immune response in the mammal that is equal to or greater than the response after delivery of the same amount of immunogenic composition by subcutaneous or intramuscular injection; the delivered immunogenic composition induces an immune response in the mammal that is equal to or greater than the response after delivery of a greater amount of the immunogenic composition by subcutaneous or intramuscular injection. Preferably, the needle is a microneedle between 0.23 and 0.025mm (31 and 50 gauge), perpendicularly inserted into the skin to a depth of between 0.3 and 1.7 mm. In one embodiment of this delivery method, an anthrax vaccine is administered as part of a prime-boost regimen.

In yet another embodiment, the invention provides an anthrax vaccine suitable for intradermal administration of an effective amount of the anthrax composition through at least one hollow needle. Preferably, the needle has an outlet with an exposed height between 0 and 1 mm, the outlet being inserted into the skin to a depth of between 0.5 mm and 2 mm. Using this and similar means of intradermal administration, the protective efficacy of an anthrax vaccine is improved.

The anthrax vaccine is suitable for eliciting (inducing) an immune response in a patient (or of vaccinating a patient against anthrax), comprising administering intradermally to the patient an effective amount of an immunogenic anthrax composition (or an anthrax vaccine). In a preferred embodiment of this method, the composition is administered through at least one hollow needle having an outlet with an exposed height between 0 and 1 mm, said outlet being inserted into the skin to a depth of between 0.5 mm and 2 mm.

The anthrax vaccine may also be suitable for topical delivery into skin, comprising abrading the skin with a solid microneedle (SMN) microabrader, and applying the immunogenic composition either before, during, or following the microabrasion step. This delivery method is sometimes referred to herein as a "topical-abrasion" method, and is described, *inter alia,* in U.S. application no. 10/282,231 published as US 2003/0094341A1.

In one embodiment an anthrax vaccine, such as an Anthrax Vaccine Absorbed (AVA) vaccine, or, preferably, a recombinant protective antigen (rPA) vaccine is delivered by pre-abrading the skin before topical application of the anthrax composition. The anthrax composition delivered may optionally comprise an adjuvant. The skin is preferably abraded at least twice, more preferably at least six times, even six to twelve times or more. The skin may be abraded in alternating directions or the same direction. The vaccine is preferably applied to the skin in liquid form, and may be applied to an abrading device, such as a coated device, or one containing a reservoir for liquids, or may be applied separately, either before or after the abrasion step. In one embodiment, a dry immunogenic composition is reconstituted simultaneously with abrasion. This may be accomplished by having the immunogenic composition present as a powder or other solid on an abrading surface of an abrading device, with a reconstituting liquid separately applied to the skin prior to abrading, or simultaneously with abrading. In one preferred embodiment, the skin is abraded using a device comprising a solid microneedle array.

In one embodiment, the skin is abraded with a device that comprises an abrading surface comprising frustoconical or frustopyrimidal microprotusions that comprise at least one scraping edge projecting from the abrading surface. In particular embodiments , the microprotrusions are at least partially coated with the anthrax vaccine; these microprotrusions being of a length sufficient to penetrate into the stratum corneum layer of the skin.

This invention also relates to kits comprising an effective amount of an anthrax vaccine as defined herein before, and, optionally means to administer the immunogenic composition intranasally.

Another embodiment of the invention is a kit for intradermal delivery of an anthrax vaccine. For example, the kit may comprise (a) a delivery device having at least one hollow microneedle designed to intradermally deliver a substance to a depth between 0.025 and 2.5 mm, said delivery device comprising or being adapted to receive a reservoir that contains an anthrax composition such that the microneedle is in communication therewith, and (b) an effective amount (dosage) of the anthrax composition. In embodiments of this kit, the dosage is contained in a reservoir that is an integral part of, or is capable of being functionally attached to, the delivery device; the hollow microneedle is between 0.23 and 0.025 mm (31 and 50 gauge); or the device comprises an array of microneedles.

In one embodiment of this kit, the device comprises (a) a hub portion being attachable to a prefillable reservoir storing the vaccine; (b) at least one microneedle supported by said hub portion and having a forward tip extending away from said hub portion; and (c) a limiter portion surrounding said microneedle and extending away from said hub portion toward said forward tip of said microneedle, said limiter including a generally flat skin engaging surface extending in a plane generally perpendicular to an axis of said microneedle and adapted to be received against the skin of a mammal to administer an intradermal injection of the anthrax composition, said microneedle forward tip extending beyond said skin engaging surface a distance approximately 0.5 mm to 2.0 mm wherein said limiter portion limits penetration of the microneedle into the dermal layer of skin of the mammal.

In another embodiment, the kit comprises (a) a dermal access means designed to intradermally deliver an anthrax composition to a depth between 0.025 and 2.5 mm, said dermal access means being adapted to receive a reservoir that contains the anthrax composition such that the dermal access means is in communication therewith, and (b) an effective amount (dosage) of the anthrax composition.

Another embodiment of the invention is a kit for delivery of an anthrax vaccine comprising use of a microabrader. For example, in one embodiment, the kit comprises (a) a microabrader comprising an abrading surface, wherein the abrading surface comprises frustoconical or frustopyramidal microprotrusions comprising at least one scraping edge and projecting from said abrading surface, and (b) an effective amount of an anthrax composition. In embodiments of this kit, the anthrax composition is coated on the surface of the microabrader; the anthrax composition is contained in a reservoir integrated with the microabrader; or the anthrax composition is separately packaged within the kit.

Yet another embodiment of the invention is a kit for the delivery of an anthrax vaccine to nasal mucosa, including the particulate or powder immunogenic anthrax composition of the invention and means for delivery. Suitable delivery devices are known in the art, for example, a single dose of medicament in powder form can be delivered intranasally using a device which allows storage of the medicament in a cartridge having burstable or pierceable membranes covering the opposed ends. Such devices can be actuated by either bursting or mechanically piercing these membranes, and expelling the medication by passage of a fluid through the cartridge. Devices of this type are described in U. S. patent application nos. 09/879,714 (filed 6/12/01) and 09/758,776 (filed 1/12/01) published as US 2002/0092524A1 and US 2002/0092523A1, respectively.

Other optional elements of a kit of the invention include suitable buffers, media components; containers; or packaging materials. The reagents of the kit may be in containers in which the reagents are stable, *e.g*., in lyophilized form or stabilized liquids. The reagents may also be in single use form, *e.g*., in single dosage form for use as vaccines.

### Anthrax compositions

Immunogenic anthrax compositions of the invention can take any suitable form. For example, the composition can comprise a mixture of several anthrax proteins, such as the mixture found in the AVA vaccine, or it can comprise isolated or purified individual components of the anthrax bacterium. Preferably, the vaccine consists essentially of just one of the anthrax proteins (*e.g*., a recombinant protein), most preferably the protective antigen (PA) protein, as described previously (Ivins, B.E., Infection and Immunity, 60:662-668, 1992; Ramirez, D.M., et al., J. Industrial Microbiol. and Biotechnol. 28:232-238, 2002 This is the anthrax protein exemplified in the Examples. The present invention is also likely to be applicable to other types of anthrax vaccines, such as AVA and DNA plasmid vaccines (e.g., Price et al., Infection and Immunity, 69:4509-4515, 2001)

A polypeptide of the invention can be a naturally occurring one or it can be produced recombinantly. (The terms polypeptide and protein are used interchangeably herein.) Preferably, a polypeptide component of the anthrax bacterium, *e*.*g*., the PA protein, is produced recombinantly. Methods to clone suitable sequences and to generate recombinant vectors in which the sequences of interest are operatively linked to suitable expression control sequences, are routine and conventional. Typical such methods (as well as molecular biology methods used in other aspects of the invention) are described in, among many other sources, Sambrook, J. et al. (1989) Molecular Cloning, a Laboratory Manual, Cold Spring Harbor Press, Cold Spring Harbor, NY, and Ausubel, F.M. et al. (1995). Current Protocols in Molecular Biology, NY, John Wiley & Sons. The phrase "expression control sequence" means a polynucleotide sequence that regulates expression of a polypeptide coded for by a polynucleotide to which it is functionally ("operably") linked. Expression can be regulated at the level of the mRNA or polypeptide. Thus, the expression control sequence includes mRNA-related elements and protein-related elements. Such elements include promoters, enhancers (viral or cellular), ribosome binding sequences, transcriptional terminators, etc. An expression control sequence is operably linked to a nucleotide coding sequence when the expression control sequence is positioned in such a manner to effect or achieve expression of the coding sequence. For example, when a promoter is operably linked 5' to a coding sequence, expression of the coding sequence is driven by the promoter. Suitable expression control sequences, such as strong constitutive or regulatable promoters, will be evident to the skilled worker.

The invention encompasses functional fragments or functional variants of wild type anthrax proteins. A "functional" fragment or variant, as used herein, includes a polypeptide that comprises any of a variety of changes (modifications), either naturally occurring or deliberately generated, provided that the polypeptide retains at least one epitope which allows it to be immunogenic. Preferably, the fragment or variant -retains the ability to elicit (induce) a protective response against anthrax infection. One of skill in the art can readily determine if a given fragment or variant exhibits the desired property, using conventional methods such as those disclosed herein. Naturally occurring allelic variants of the proteins are encompassed by the invention.

Polypeptides used in compositions or methods of the invention can be functional fragments of full-length proteins. Any desirable size (length) polypeptide can be used, ranging from one amino acid shorter than a wild type protein to a small peptide (*e*.*g*., 8-10 amino acids long) bearing only a single epitope and/or antigenic sequence.

The polypeptides can also be functional variants. For example, variant PA polypeptides used in the methods of the invention may exhibit substantial identity to comparable portions of wild type PA. The term "substantial identity" or "substantial similarity" as used herein indicates that a polypeptide (or a nucleic acid) comprises a sequence that has at least 90% sequence identity to a reference sequence, or preferably at least 95%, or more preferably at least 98% sequence identity to the reference sequence, over a comparison window of at least 10 to 100 or more amino acids residues or nucleotides. Methods to determine sequence identity (between nucleic acids or proteins) are conventional. Alignments can be accomplished by using any effective algorithm. For pairwise alignments of DNA sequences, the methods described by Wilbur-Lipman (*e.g*., Wilbur et al. (1983), Proc. Natl. Acad Sci., 80, 726-730) or Martinez/Needleman-Wunsch (*e.g.,* Martinez (1983), Nucleic Acid Res. 11, 4629-4634) can be used. Pairs of protein sequences can be aligned by the Lipman-Pearson method (*e.g.,* Lipman et al. (1985), Science 227,1435-1441), *e.g.,* with k-tuple set at 2, gap penalty set at 4, and gap length penalty set at 12. Various commercial and free sources of alignment programs are available, *e.g*., MegAlign by DNA Star, BLAST (National Center for Biotechnology Information), BCM (Baylor College of Medicine) Launcher, etc. Percent sequence identity can also be determined by other conventional methods, *e.g.,* as described in Altschul et al.(1986), Bull. Math. Bio. 48, 603-616, 1986 and Henikoff et al. (1992), Proc. Natl. Acad. Sci. USA 89,10915-10919.

Variant polypeptides of the invention include polypeptides having one or more naturally occurring (*e.g.,* through natural mutation) or non-naturally-occurring (*e.g.,* by deliberate modification, such as by site-directed mutagenesis) modifications, *e*.*g*., insertions, deletions, additions and/or substitutions, either conservative or non-conservative. By "conservative substitutions" is meant by combinations such as Gly, Ala; Val, Ile, Leu; Asp, Glu; Asn, Gln; Ser, Thr; Lys, Arg; and Phe, Tyr. Variants can include, *e.g*., homologs, muteins and mimetics. Many types of protein modifications, including post-translational modifications, are included. Post-translational modifications include naturally occurring or synthetically produced, covalent or aggregative conjugates with other chemical moieties, *e*.*g*., glycosyl groups, lipids, phosphates, acetyl groups, etc., as well as cleavage, such as of terminal amino acid(s). See, *e.g*., modifications disclosed in U.S. Pat. No. 5,935,835. The invention also encompasses variants such as polypeptides in which cysteine residues which are nonessential for biological activity are deleted or replaced with other amino acids, thereby preventing the formation of incorrect intramolecular disulfide bridges; naturally occurring variants arising from alternative mRNA splicing events; and altered forms reflecting genetic polymorphism (*e*.*g*., allelic variation). Other active variants may comprise added peptide sequences, either naturally occurring or heterologous, such as, *e*.*g*., leader, signal, secretory, targeting and enzymatic sequences. Furthermore, the polypeptide can be modified by any of a variety of art-recognized modifications, such as in the variant polypeptides discussed in US2002/0052475.

### Compositions and methods for intranasal (IN) delivery

One aspect of the present invention relates to methods of preparing dried pharmaceutical compositions, in particulate form (*e.g*., in a powder), which are suitable for direct intranasal administration, and to compositions made by these methods. Such dried particles can also be reconstituted in a pharmaceutically acceptable liquid (aqueous) form prior to delivery to mucosa or to skin.

As noted above, one aspect of the invention is a method of preparing an immunogenic anthrax composition (*e.g*., an anthrax vaccine), comprising one or more of the following steps: atomizing a liquid formulation of an anthrax composition to produce an atomized formulation; freezing said atomized formulation to form solid particles; and drying said solid particles to produce dried particles. Preferably, said atomized formulation comprises droplets having a volume mean diameter (as defined by W.H.Finley, "The mechanics of inhaled pharmaceutical aerosols, an introduction", Academic Press, London, UK (2001)) of between 35 µm and 300 µm. In a preferred embodiment, the powder comprises dried particles that have a mean aerodynamic diameter (as defined in W.H. Finley, *supra*) of between 8 µm and 140 µm. This method, and compositions made by the method, are sometimes generally referred to herein as a "spray-freeze-dry" (SFD) method or compositions.

Particles of the above pharmaceutical compositions are of an appropriate size, density and/or morphology to facilitate intranasal administration. Without wishing to be bound by any particular theory, it is proposed that, following intranasal administration, the compositions described above are delivered to mucous membranes, *e.g*., of the nasal lining or the sinuses, where they adhere, rather than being propelled through the sinus cavities into the pulmonary system, and that adherence to such mucous membranes allows a faster rate of absorption than with other formulations of therapeutic and prophylactic compositions. When the inventive composition is a vaccine, the time required to mount an effective antibody response is thus reduced, and mucosal immunity (*e.g*., mediated by an IgA response) is enhanced. Intranasal administration of such inventive vaccines elicits both systemic and naso-mucosal IgA immune responses.

As noted above, another aspect of the invention is a method of preparing an immunogenic anthrax composition (*e.g*., an anthrax vaccine), comprising one or more of the following steps: atomizing a liquid formulation of an anthrax composition to produce an atomized formulation; freezing said atomized formulation to form solid particles; and drying said solid particles at about atmospheric pressure, in the presence of vibration, internals, mechanical stirring, or a combination thereof, to produce dried particles (*e*.*g*.. to produce a powder). By "about atmospheric pressure" is meant a pressure ranging from about one half atmosphere to about five atmospheres. By "drying" is meant removal of the volatile components of the formulation from the solid frozen particles. Preferably, the powder comprises dried particles having a volume mean diameter of between 35µm and 300µm, more preferably between 50µm and 300µm and/or the dried particles have a volume mean aerodynamic diameter of between 8µm and 140µm. Preferably, the frozen, solid particles are in a fluidized state as they are being dried. This method, and compositions made by the method, are sometimes referred to herein as "spray-freeze-atmospheric-dry" method or compositions. An advantage of compositions produced by this method is that they do not agglomerate.

A further discussion of "spray-freeze-dried" and "spray-freeze-atmosphere-dried" methods and compositions can be found in U.S. patent application nos. 10/299,012 and 10/299,010 published as US 2003/0180755A1 and US 2003/0186271A1, respectively.

Additional advantages of the compositions of the invention are that the compositions are readily aerosolized, offer good stability, sterility, emitted dose and preservation of bioactivity, leave little residue in a delivery device such as an inhalation device, and are readily reconstituted in liquid. The particles may exhibit a low tap density, which facilitates the aerosolization of the particles, *e.g*., for respiratory delivery. The particles exhibit low levels of fines, which renders them easy to handle. The methods of the invention allow for a well-controlled distribution of particle sizes. Therefore, the inventive compositions comprise a well-controlled distribution of particle sizes. The morphology of the particles reduces the potential for their agglomerization and further facilitates aerosolization. The compositions are stable in the absence of refrigeration, allowing for more convenient and less expensive storage and transportation than, *e.g*., liquid formulations. Compositions of the invention are particularly well suited for mass vaccinations. In one embodiment of the invention, inventive compositions are administered by respiratory (*e.g*., intranasal) administration. Compositions of the invention are particularly well suited to intranasal administration, since the well controlled particle size distribution allows accurate targeting of the nasal mucosa. Advantages of respiratory administration compared to administration by injection (*e.g*., intradermal or sub-dermal) include increased patient compliance and enhanced immune responses. Respiratory administration is also advantageous in that it is a non-invasive procedure, which is painless and easy to perform.

An immunogenic composition of the invention can be initially formulated as a liquid formulation, using any of a variety of conventional liquids. Preferably, the liquid is an aqueous one, such as, *e.g*., water (*e*.*g*., injectable grade water) or any of a variety of conventional buffers, which may or may not contain additional salts. The pH of the buffer will generally be chosen to stabilize the protein, and will be ascertainable by those in the art. Generally, this will be in the range of physiological pH. Thus, preferred pH ranges of the initial liquid formulation are from 1 to 10, with from 3 to 8 being particularly preferred, and from 5 to 7 being especially preferred. As will be appreciated by those in the art, there are a large number of suitable buffers that may be used. Suitable buffers include, but are not limited to, sodium acetate, sodium citrate, sodium succinate, ammonium bicarbonate and carbonate. Generally, buffers are used at molarities from 1 mM to 2 M, with from 2 mM to 1 M being preferred, and from 10 mM to 0.5 M being especially preferred, and 50 to 200 mM being particularly preferred. Generally, salts, if present in the liquid solution, are used at molarities from 1 mM to 2 M, with from 2 mM to 1 M being preferred, and from 10 mM to 0.5 M being especially preferred, and 50 to 200 mM being particularly preferred. Suitable salts include, but are not limited to, NaCl.

The liquid formulation can be in any of a variety of forms, *e.g*., a solution, a suspension, a slurry or a colloid. Optionally, the liquid formulation can comprise one or more conventional pharmaceutically acceptable excipients. "Excipients" generally refer to compounds or materials that are added to enhance the efficacy of a formulation of an active pharmaceutical ingredient (API). Examples include, *e.g*., cryoprotectants and lyoprotectants, which are added to ensure or increase the stability of the protein during the spray-freeze dry process or spray-freeze atmosphere dry process, and afterwards, for long term stability and flowability of the powder product. Suitable protectants are generally relatively free flowing particulate solids, do not thicken or polymerize upon contact with water, are essentially innocuous when inhaled by a patient or otherwise introduced into a patient, and do not significantly interact with the therapeutic agent in a manner that alters its biological activity. Suitable excipients include, but are not limited to, proteins such as human and bovine serum albumin, gelatin, immunoglobulins, carbohydrates including monosaccharides (galactose, D-mannose, sorbose), disaccharides (lactose, trehalose, sucrose), cyclodextrins, and polysaccharides (raffinose, maltodextrins, dextrans); an amino acid such as monosodium glutamate, glycine, alanine, arginine or histidine, as well as hydrophobic amino acids (tryptophan, tyrosine, leucine, phenylalanine); a methylamine such as betaine; an excipient salt such as magnesium sulfate; a polyol such as trihydric or higher sugar alcohols, e.g. glycerin, erythritol, glycerol, arabitol, xylitol, sorbitol, and mannitol; propylene glycol; polyethylene glycol; Pluronics; surfactants; and combinations thereof. Preferred excipients include *e.g*., trehalose, sucrose and mannitol. Another class of excipient, mucoadhesives, are often used to increase contact of an API with mucosal surfaces. Examples of mucoadhesives include, *e.g*., chitosan, dermatan sulfate, chondroitin, and pectin. Additionally, conventional cosolvents, which improve the solubility of APIs, can be added to liquid formulations suitable for the SFD processes disclosed herein.

Generally, when mucoadhesives are used, they are used in amounts ranging from 1 to 95 wt %, with from 1 to 50 wt % preferred, from 5 to 50 wt % being especially preferred, and from 5 to 20% being particularly preferred. In general, cryoprotectants are used at a concentration of between 5 wt% and 95 wt%.

In one embodiment, the dried powders of the invention are later combined with bulking agents or carriers, which are used to reduce the concentration of the therapeutic agent in the powder being delivered to a patient; that is, it may be desirable to have larger volumes of material per unit dose. Bulking agents may also be used to improve the dispersibility of the powder within a dispersion device, and/or to improve the handling characteristics of the powder. This is distinguishable from the use of bulking agents or carriers during the spray-drying process. Suitable bulking agents are generally crystalline (to avoid water absorption) and include, but are not limited to, lactose and mannitol. Accordingly, bulking agents such as lactose, if added, may be added in varying ratios, with from 99:1 of a therapeutic agent of interest to bulking agent to 1:99 being preferred, and from 1:5 to 5:1 being more preferred, and from 1:10 to 1:20 being especially preferred.

Liquid formulations of the invention can be atomized by any of a variety of conventional procedures. For example, the liquid can be sprayed through a two-fluid nozzle, a pressure nozzle, or a spinning disc, or atomized with an ultrasonic nebulizer or a vibrating orifice aerosol generator (VOAG). In one embodiment, a liquid formulation is atomized with a pressure nozzle such as a BD AccuSpra^{™} nozzle.

In a preferred embodiment, atomization conditions are optimized such that the mean mass diameter of the atomized droplets (*e.g*., nebulized droplets) is at least 20µ, preferably between 35µm and about 300µm, more preferably between 50µm and 300µm, still more preferably between 50µm and 100µm. Methods to optimize the generation of droplets of the desired size are conventional. Among the conditions that can be varied are atomization gas flow, atomization gas pressure and liquid flow rate. Also, the type and size of the nozzle can be varied. Liquid drop size can be readily measured, using conventional techniques, such as laser diffraction. The size of dried particles can be measured by conventional techniques, such as, *e.g*., scanning electron microscopy (SEM) or laser diffraction.

In one embodiment, in which frozen, atomized particles are dried at about atmospheric pressure, as is discussed elsewhere herein, the size of the atomized droplets can be, *e.g*., at least about 20µm, preferably between 20µm and 300µm, more preferably between 35µm and 100µm or between 50µm and 100µm.

Following the atomization of a liquid formulation, the droplets are rapidly frozen to form solid particles. Preferably, the droplets are frozen immediately, or substantially immediately, after the atomization step.

In one embodiment, the droplets are frozen by immersing them in a cold liquid that is below the freezing point of the liquid formulation from which the atomized droplets were formed. In a preferred embodiment, the temperature of the cold liquid is -200° C to -80° C, more preferably between -200° C to -100° C, most preferably -200° C (liquid nitrogen is -196° C). Any suitable cold liquid may be used, including liquid nitrogen, argon and hydrofluoroethers, or a compressed liquid, such as compressed fluid CO₂, helium, propane or ethane, or equivalent inert liquids, as is well known in the art. For example, in one embodiment, a liquid preparation of an anthrax composition is atomized through a spray nozzle that is positioned above a vessel containing a suitable cold liquid, such as, *e.g*., liquid nitrogen. The droplets freeze instantaneously upon contact with the cold liquid.

In another embodiment, the droplets are frozen by passage through a gas (*e.g*., cold air, nitrogen, helium or argon), in a cooling chamber, wherein the gas is below the freezing point of the droplets. In a preferred embodiment, the cold gas is -5° C to -60° C, more preferably between - 20° C to -40° C. The gas can be cooled by conventional methods, such as by cooling coils, heat exchangers or chiller condensers. The temperature of the gas can be reduced with conventional procedures, *e.g*., with liquid nitrogen, solid carbon dioxide or an equivalent cryogenic agent to produce the subfreezing temperatures.

Following the formation of solid frozen particles, the particles are dried to produce a powder. By "dry" is meant having a negligible amount of liquid, *e.g*., having a moisture content such that the particles are readily dispersible to form an aerosol, *e.g*. in an inhalation device. This moisture content is generally below 15% by weight water, with less than 10% being preferred and less than 1% to 5% being particularly preferred.

In one embodiment of the invention, the frozen droplets are dried by lyophilization (freeze-drying, under vacuum), using a conventional lyophilization apparatus. This method is generally called a "spray-freeze-dry" or SFD method, and compositions made by the method are called "spray-freeze-dry" or SFD compositions. For example, in one embodiment, when particles have been frozen by spraying them into a vessel (such as a Virtis freeze-drying flask) containing liquid nitrogen, the vessel can then be attached to a conventional lyophilizer and the excess liquid nitrogen evaporated off. The frozen aerosol is typically dried within about 48 hours and reaches a moisture level below about 1 wt%. Alternatively, droplets that have been frozen in cold air at about atmospheric pressure and, optionally, partially dried at about atmospheric pressure (as is discussed below) can then be placed in a lyophilization flask and subjected to lyophilization.

In another embodiment, the frozen droplets are dried by sublimation in a cold, desiccated gas (*e.g*., air, nitrogen or helium) stream at about atmospheric pressure. By "about atmospheric pressure" is meant herein a pressure ranging from about one half atmosphere to about five atmospheres. The temperature of the gas can be reduced by any of a variety of conventional procedures, *e.g*., with liquid nitrogen, solid carbon dioxide or an equivalent cryogenic agent. Particles of the invention that are dried in such a manner are sometimes referred to herein as "spray-freeze-atmosphere-dried" particles. In a preferred embodiment, atomized droplets are frozen and dried in the same "spray-freeze-atmosphere-dry" chamber, allowing the freezing and drying procedures to be carried out in a single step.

One apparatus and method for drying solid, frozen particles in cold air at about atmospheric pressure is disclosed in Leuenberger, USP 4,608,764. See also Examples in U.S. patent application nos. 10/299,012 and 10/299,010 published as US 2003/0180755A and US 2003/0186271A1, respectively Other types of conventional apparatus can also be used, as will be appreciated by persons of skill in the art.

In a preferred embodiment, frozen atomized particles are dried in a cold gas at about atmospheric pressure in the presence of conditions that enhance fluidization of the particles. In a most preferred embodiment, the frozen, atomized particles are dried in the presence of vibration, internals, mechanical stirring, or combinations thereof, during the drying process. The term, "intemals," as used herein, refers to any physical barrier inside a chamber (*e.g*., the SFD chamber) or fluidized bed, such as, *e.g*., blades, plates or other barriers. Such treatments allow the particles to achieve a fluidized state, and help to prevent channeling.

In another embodiment, the frozen droplets are dried by a combination of sublimation in a cold, desiccated gas (*e.g*., air) stream at about atmospheric pressure, as described above, and lyophilization. For example, a composition that has been partially dried at about atmospheric pressure (*e.g*., to form a cake or a powder that still contains undesirable amounts of liquid) is removed to a lyophilizer, in which the composition is dried further.

Conventional methods can be used to collect the dried compositions. In one embodiment, the dried particles are collected on a filter, from which they can be removed for use in, *e.g*., medical applications. In another embodiment, the spray-freeze atmosphere dried particles are collected in a product vessel. Partially dried particles may form a loose cake, from which remaining moisture can be removed by further atmospheric sublimation in a cold desiccated air stream, or they can optionally be removed to a lyophilizer or other suitable device and further dried under reduced pressure (below atmospheric pressure.)

Particles dried by any of the above methods exhibit substantially the same properties (*e.g*., particle size and porosity).

Following the drying procedure, a composition of the invention can achieve the form of a free-flowing powder. The dry, porous particles of the composition are roughly the same size (geometric diameter) and shape as the frozen droplets prior to drying.

Dried particles of the invention exhibit desirable aerodynamic properties show a desirable morphology (as shown by SEM) and a desirable density. Furthermore, the particles exhibit lower amounts of residual powder remaining in a delivery device following its use, exhibit greater stability than, *e.g*., liquid insulin formulations, and are readily reconstituted in liquid.

One aspect of the invention is the use of an immunogenic anthrax composition produced by a method of the invention for preparing anthrax vaccine, which is suitable to administer an effective amount of anthrax to a patient. By an "effective amount" is meant herein an amount that is effective to elicit a desired response. For example, an effective amount of an immunogenic composition is an amount that is effective to elicit a detectable immune response. An effective dose can be determined empirically, according to conventional procedures, taking into account well known factors such as the age, weight, and/or clinical condition of the patient, the method of and scheduling of administration. As for the vaccine disclosed herein, the patient can be any animal, preferably a mammal such as, *e.g*., a farm or other domestic animal, or a rat, mouse, hamster, guinea pig or rabbit, preferably a human.

The vaccines of the invention are suitable to be administered by any of a variety of routes that are known to the skilled worker, including respiratory preferably intranasal, routes. In one embodiment, the vaccine is suitable to be administered to a mucosal tissue, such as a mucosal tissue of the nasal passages and the sinuses. In a preferred embodiment, the vaccine of the invention is suitable to be administered to a patient in need thereof via the respiratory system. By "administration through the respiratory system" or "respiratory administration" is meant herein that an agent is administered to the nose (intranasally), or the lungs (pulmonary administration).

Conventional methods of administration may be used. Suitable applicators (*e.g*., inhalers) are known in the art. Typical delivery devices include, but are not limited to, the devices disclosed in U. S. patent application no. 09/879,714 (filed 6/12/01) and 09/758,776 (filed 1/12/01) published as US 2002/0092524A1 and US 2002/0092523A1, respectively. In a preferred embodiment, the device used for nasal administration is a "Solovent" apparatus. Dosages to be administered can be determined by conventional procedures known to those of skill in the art. See, *e.g.,* The Pharmacological Basis of Therapeutics, Goodman and Gilman, eds., Macmillan Publishing Co., New York. Factors to be considered include the activity of the specific agent involved, the metabolic stability and length of action of the agent, mode and time of administration, drug combination, rate of excretion, the species being treated, and the age, body weight, general health, sex, diet and severity of the particular disease states of the host undergoing therapy. Dosages for eliciting an effective immune response (*e.g*., dosages for effective vaccination) are well known to those of skill in the art.

The invention also includes a unit dosage receptacle or dry powder inhaler, comprising an effective amount of an immunogenic anthrax composition (*e.g*., an anthrax vaccine) of the invention.

The vaccineCompositions of the invention elicits a greater therapeutic effect following intranasal administration than do liquid formulations. See, *e.g*., Example 11. Therefore, it is possible to reduce the amount of immunogenic anthrax composition in the anthrax vaccine that is required to produce an efficacious result following intranasal administration to a patient in need thereof.

The vaccine of the invention elicits (induces) an immune response in a patient, after being administered to the patient. The term "immune response" as used herein encompasses, for example, mechanisms by which a multi-cellular organism produces antibodies against an antigenic material that invades the cells of the organism or the extra-cellular fluid of the organism. The antibody so produced may belong to any of the immunological classes, such as immunoglobulins A, D, E, G or M. Other types of responses, for example cellular immunity, such as the induction of cytotoxic T cells, are also included. Immune response to antigens is well studied and widely reported. A survey of immunology is given, *e.g*., in Roitt I., (1994). Essential Immunology, Blackwell Scientific Publications, London. Methods in immunology are routine and conventional (see, *e.g.,* Currents Protocols in Immunology; edited by John E. Coligan et al., John Wiley & Sons, Inc.).

In one aspect, the immunogenic anthrax composition is an anthrax vaccine (a vaccine used to stimulate the immune system of a living organism so that protection against future infection by anthrax is provided). That is, the vaccine can result in the amelioration of at least some of the symptoms engendered by infection with anthrax. The delivery platforms and methods described herein would also be applicable to gene therapy methods of introducing nucleic acids encoding the anthrax PA.

Inhalation anthrax enters the body and exerts its pathophysiologic effects through nasal mucous membranes. Protection against infection can be provided at the first site of entry by neutralizing infectious agents with locally produced mucosal IgA. Although systemic immune responses are readily elicited by traditional immunization routes (such as IM, ID), mucosal responses are more difficult to achieve in general. An advantage of intranasal (IN) delivery of dry powder vaccine is its ability to elicit both systemic and mucosal immune response. In addition, IN powder vaccine delivery may reduce the required dose because of the effectiveness of mucosal powder drug intake.

### Intradermal (ID) delivery methods, using hollow microneedles (HMN) or solid microneedles (SMN)

In one aspect, the present invention utilizes "microneedles", both "hollow" and "solid," to administer an anthrax rPA vaccine to skin. According to the present invention, "hollow" microneedles are small microprojections that enable reproducible intra-dermal (ID) delivery. Devices comprised of such microneedle structures and suitable fluid-driving mechanisms are known in the art. Suitable "hollow" microneedle structures and fluid-driving mechanisms are described in detail in U.S. applications no. 10/028,988, filed December 28, 2001 published as US 2003/0100885A1 and 10/185,717, filed July 1, 2002 published as US 2002/0198509A1, and WO 02/02179 A1.

Delivery with hollow microneedles (HMN) allows delivery into an intradermal space within mammalian skin. An immunogenic anthrax composition can be administered, *e.g*., through a small gauge hollow needle having an outlet with an exposed height between 0 and 1 mm, said outlet being inserted into the skin to a depth of between 0.3 mm and 2 mm, such that delivery of the substance occurs at a depth between 0.3 mm and 2 mm. It has been found that using the method of the invention, the effective amount is less than that required to be administered by intramuscular injection to achieve an immunogenic effect, such as protective immunity.

One benefit of the invention is that ID administration with enhanced immunogenicity allows equivalent biological effects while using less of an immunogenic composition. This results in direct economic benefit to the manufacturer and perhaps consumer, especially for expensive vaccine preparation. Likewise, higher immunogenicity allows reduced overall dosing and decreases patient side effects associated with higher dosing.

Anatomically, the outer surface of the body is made up of two major tissue layers, an outer epidermis and an underlying dermis, which together constitute the skin (for review, see Physiology, Biochemistry, and Molecular Biology of the Skin, Second Edition, L.A. Goldsmith, Ed., Oxford University Press, New York, 1991). The epidermis is subdivided into five layers or strata of a total thickness of between 75 and 150 µm. Beneath the epidermis lies the dermis, which contains two layers, an outermost portion referred to at the papillary dermis and a deeper layer referred to as the reticular dermis. The papillary dermis contains vast microcirculatory blood and lymphatic plexuses. In contrast, the reticular dermis is relatively acellular and avascular and made up of dense collagenous and elastic connective tissue. Beneath the epidermis and dermis is the subcutaneous tissue, also referred to as the hypodermis, which is composed of connective tissue and fatty tissue. Muscle tissue lies beneath the subcutaneous tissue.

It is appreciated by those of skill in the art that skin thickness varies, both among individuals, and in any individual between different locations on the body. Thus, depending on the location of administration, the depth of delivery needed to target the ID space will vary.

The subject of intradermal delivery of the present invention is a mammal, preferably, a human. The biologically active reagent, with or without a tracer reagent, may be delivered into the intradermal space by a needle or cannula, usually from 300 µm to 5 mm long. Preferably, the needle or cannula is 300 µm to 1 mm long, with the outlet inserted into the skin of the subject to a depth of 1 mm to 3 mm. Preferably, a small gauge needle or cannula, between 0.25 and 0.13 mm (30 and 36 gauge), preferably 0.23 and 0.16 mm (31-34 gauge) is used. The outlet of the needle or cannula is preferably inserted to a depth of 0.3 mm (300 µm) to 1.5 mm.

The immunogenic anthrax composition may be infused, or preferably, administered as a bolus. As used herein, the term "bolus" is intended to mean an amount that is delivered within a time period of less than ten (10) minutes. "infusion" is intended to mean the delivery of a substance over a time period greater than ten (10) minutes It is understood that bolus administration or delivery can be carried out with rate controlling means, for example a pump, or have no specific rate controlling means, for example user self-injection.

In one preferred embodiment, an immunogenic anthrax composition is administered through at least one small gauge hollow needle having an outlet with an exposed height between 0 and 1 mm, said outlet being inserted into the skin to a depth of between 0.3 mm and 2 mm, such that delivery of the composition occurs at a depth between 0.3 mm and 2 mm.

As used herein, "intradermal delivery" means the delivery of materials to the intradermal space and its interrogation as described by Pettis et al. in WO 02/02179 having a priority date of June 29, 2000.

In a particularly preferred embodiment the immunogenic composition is delivered though a device that allows skilled and unskilled medical staff to reach the dermal space with each attempt. Micro-cannula- and microneedle-based methodology and devices are described in WO 02/02178A1/WO 02/02179A1 claiming priority of U.S. Application Serial No. 606,909, filed June 29, 2000. Standard steel cannula can also be used for intra-dermal delivery using devices and methods as described in EP 1092444B1 claiming priority of U.S. Serial No. 417,671, filed October 14, 1999. These methods and devices include the delivery of substances through narrow gauge (30G or narrower) "micro-cannula" with a limited depth of penetration (typically ranging from 10 µm to 2 mm), as defined by the total length of the cannula or the total length of the cannula that is exposed beyond a depth-limiting hub feature. In one preferred embodiment, the microneedle is configured in a delivery device which positions the microneedle perpendicular to skin surface.

As used herein, "conventional delivery" means any method for delivering any material that has, or is thought to induce immune responses that are similar in magnitude or weaker than via intramuscular delivery. Conventional delivery may include subcutaneous, iontophoretic, and intradermal delivery methods such as those described in US 5,800,420 to Gross.

According to the present invention, "solid" microneedles (SMN) are small microprojections that are used to abrade the stratum corneum to allow the vaccine to access the epidermal tissue. The microprojections are part of an "abrading surface" that, together with a suitable base and handle, make up a "microenhancer" or "microabrader" device. These devices are typically used in association with topical vaccine delivery to the skin. Preferably, the device used in the present invention penetrates, but does not pierce, the stratum corneum. The substance to be administered using the methods of this invention may be applied to the skin prior to abrading, simultaneous with abrading, or post-abrading. Devices comprised of a suitable abrading surface, base and handle are referred to herein as SMN devices. Suitable such devices are known in the art and are described for example in WO 01/39693A2 claiming priority of U.S. application nos. 09/405,488, filed September 24, 1999; and WO 01/89622A1 claiming priority of 09/576,643, filed May 22, 2000; 09/974,829, filed October 12, 2001 published as US 2002/0053756A1; and 10/282,231, filed October 29, 2002 published as US 2003/0093040A1.

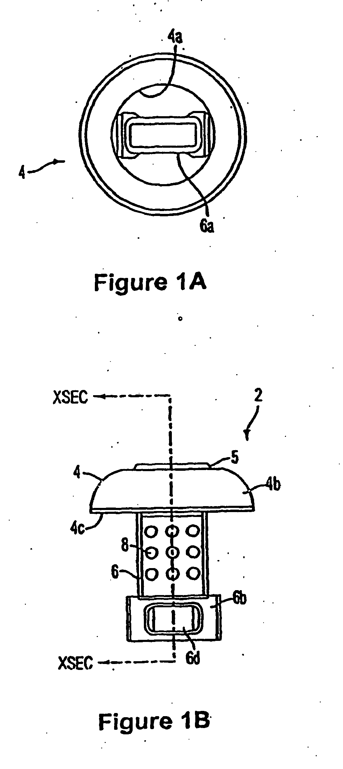

The SMN method for delivering a vaccine or other substance includes the steps of coating a patient's outer skin layer or a microabrader 2 , see Figure 1B with a medicament or other substance and moving microabrader 2 across the patient's skin to provide abrasions leaving furrows sufficient to permit entry of the substance into the patient's viable epidermis. Due to the structural design of microabrader 2, the leading edge of microabrader 2 first stretches the patient's skin and then the top surface of microabrader 2 abrades the outer protective skin layer opening the stratum corneum thereby permitting medicament or other substance to enter the patient. After the initial abrasion of the outer protective skin layer, the trailing and leading edges of microabrader 2 can rub the surface of the abraded area working the medicament or substance into the abraded skin area thereby improving its medicinal effect.

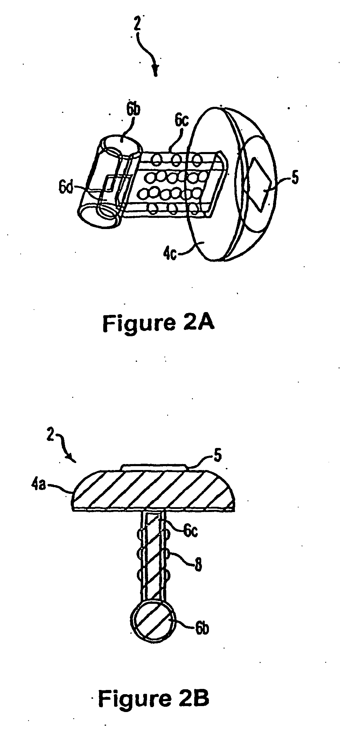

As shown in Figures 1B, 2A and 2B, microabrader 2 includes base 4 onto which an abrading surface 5 can be mounted. Alternatively, the abrading surface may be integral with the base and fabricated as a single two-component part. Preferably, base 4 is a solid molded piece. In one embodiment, base 4 is configured with a mushroom-like crown 4b that curves upward and is truncated at the top. The top of base 4 is generally flat with abrading surface 5 being mounted thereon or integral therewith. Alternatively, the truncated top may have a recess for receiving abrading surface 5. In all embodiments, abrading surface 5 includes a platform with an array of microprotrusions that extends above the truncated top. In another embodiment of the microabrader, the handle, base and abrading surface may be integral with one another and fabricated as a single three-component device.

Microabrader 2 is applied to a subject by moving microabrader 2 across the subject's skin with enough pressure to enable abrading surface 5 to open the outer protective skin or stratum corneum of the subject. The inward pressure applied to the base causes microabrader 2 to be pressed into the subject's skin. Accordingly, it is preferable that the height of the sloping mushroom-like crown 4b be sufficient to prevent the applied substance from flowing over and onto the facet 4c when microabrader 2 is being used. As will be described below, abrading surface 5 comprises an array of microprotrusions.

A handle 6 is attached to base 4 or may be integral with base 4. As shown in Fig. 2A, an upper end 6a of the handle may be either snap fit or friction fit between the inner circumferential sidewall 4a of base 4. Alternatively, as shown in Figures 1A and 2A, handle 6 may be glued (*e.g*., with epoxy) to the underside 4c of base 4. Alternatively, the handle and base may be fabricated (*e.g*., injection-molded) together as a single two-component part. The handle may be of a diameter that is less than the diameter of the base or may be of a similar diameter as the base. Underside 4c of base 4 may be flush with mushroom-like crown 4b or extend beyond the mushroom-like crown. The lower end 6b of handle 6 may be wider than the shaft 6c of handle 6 or may be of a similar diameter as shaft. Lower end 6b may include an impression 6d that serves as a thumb rest for a person administering the substance and moving microabrader 2. In addition, protrusions 8 are formed on the outside of handle 6 to assist a user in firmly gripping handle 6 when moving the same against or across a patient's skin.

As shown in the cross-section of Figure 1B in Figure 2B, lower end 6b may be cylindrical. Microabrader 2 may be made of a transparent material, as shown in Figure 2A. Impressions 6d are disposed on both sides of the cylindrical lower end 6b to assist a person using microabrader 2 to grip the same. That is, the movement of microabrader 2 can be provided by hand or fingers. The handle 6, as well as the base 4, of the microabrader is preferably molded out of plastic or the like material. The microabrader 2 is preferably inexpensively manufactured so that the entire microabrader and abrading surface can be disposed after its use on one patient.

Abrading surface 5 is designed so that when microabrader 2 is moved across a patient's skin, the resultant abrasions penetrate the stratum corneum. Abrading surface 5 may be coated with a medicament or substance desired to be delivered to the patient's viable epidermis.

In order to achieve the desired abrasions, the microabrader 2 should be moved across a patient's skin at least once. The patient's skin may be abraded in alternating directions. The structural design of the microabrader according to the invention enables the medicament or substance to be absorbed more effectively thereby allowing less of the medicament or substance to be applied to a patient's skin or coating abrading surface 5.

Abrading surface 5 may be coated with a substance desired to be delivered to the patient. In one embodiment, the substance may be a powder disposed on abrading surface 5. In another embodiment, the substance to be delivered may be applied directly to the patient's skin prior to the application and movement of microabrader 2 on the patient's skin.

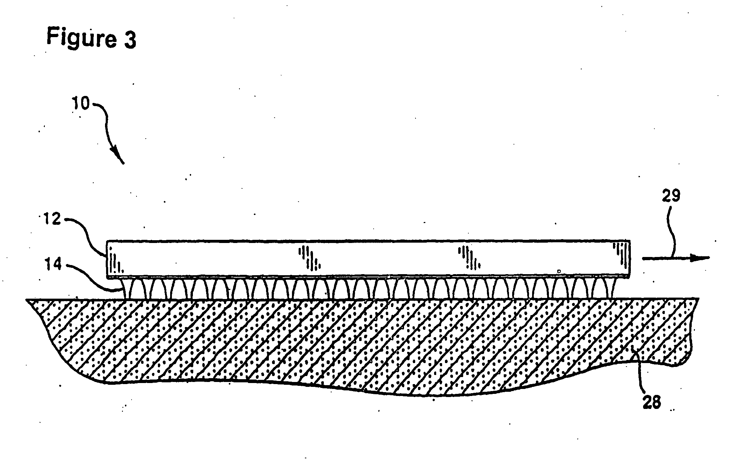

Referring to Figure 3, the microabrader device 10 of the invention includes a substantially planar body or abrading surface support 12 having a plurality of microprotrusions 14 extending from the bottom surface of the support. The support generally has a thickness sufficient to allow attachment of the surface to the base of the microabrader device thereby allowing the device to be handled easily as shown in Figure 1B, 2A and 2B. Alternatively, a differing handle or gripping device can be attached to or be integral with the top surface of the abrading surface support 12. The dimensions of the abrading surface support 12 can vary depending on the length of the microprotrusions, the number of microprotrusions in a given area and the amount of the substance to be administered to the patient. Typically, the abrading surface support 12 has a surface area of about 1 to 4 cm². In preferred embodiments, the abrading surface support 12 has a surface area of 1 cm².

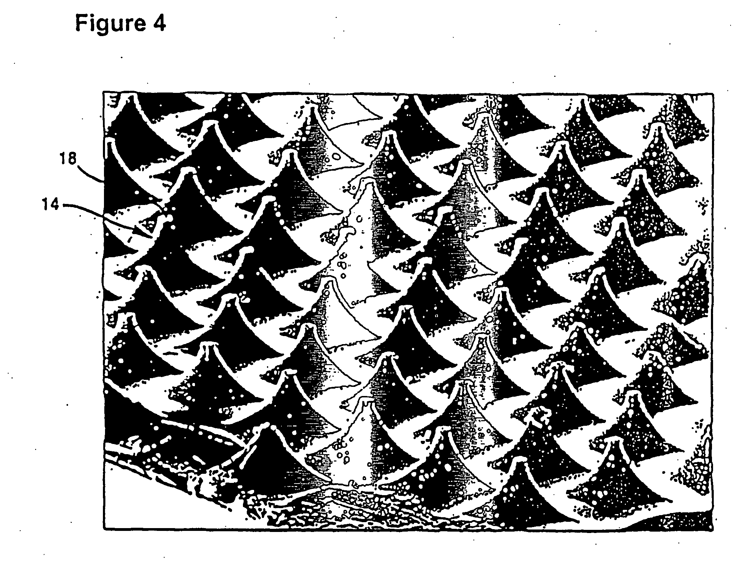

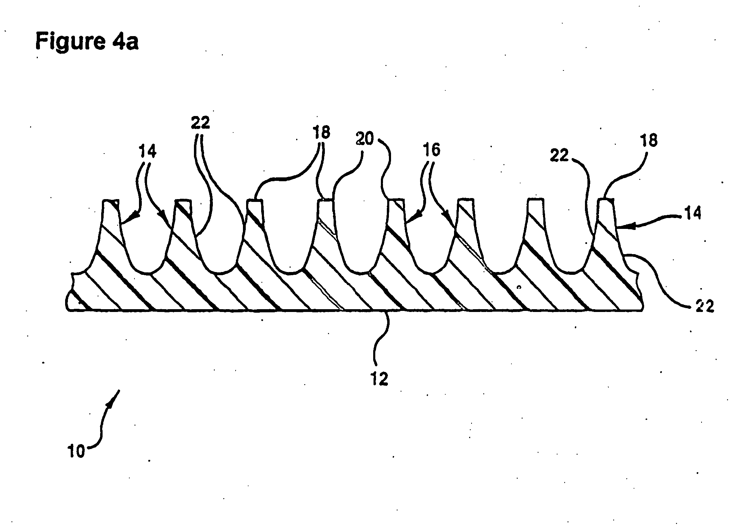

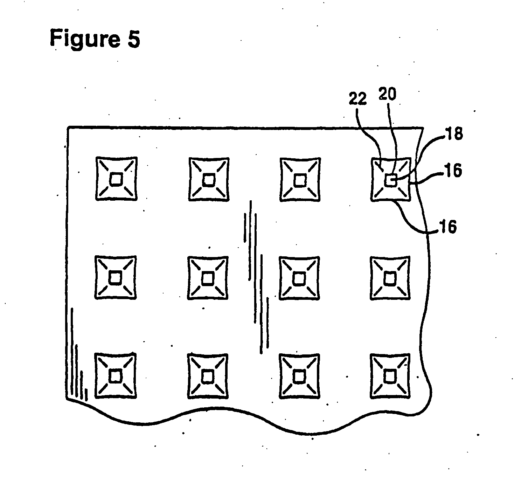

As shown in Figures 3, 4, 4A and 5, the microprotrusions 14 project from the surface of the abrading surface support 12 and are substantially perpendicular to the plane of the abrading surface support 12. The microprotrusions in the illustrated embodiment are arranged in a plurality of rows and columns and are preferably spaced apart a uniform distance. The microprotrusions 14 have a generally pyramid shape with sides 16 extending to a tip 18. The sides 16 as shown have a generally concave profile when viewed in cross-section and form a curved surface extending from the abrading surface support 12 to the tip 18. In the embodiment illustrated, the microprotrusions are formed by four sides 16 of substantially equal shape and dimension. As shown in Figures 4A and 5, each of the sides 16 of the microprotrusions 14 have opposite side edges contiguous with an adjacent side and form a scraping edge 22 extending outward from the abrading surface support 12. The scraping edges 22 define a generally triangular or trapezoidal scraping surface corresponding to the shape of the side 16. In further embodiments, the microprotrusions 14 can be formed with fewer or more sides.

The microprotrusions 14 preferably terminate at blunt tips 18. Generally, the tip 18 is substantially flat and parallel to the support 14. When the tips are flat, the total length of the microprotrusions do not penetrate the skin; thus, the length of the microprotrusions is greater than the total depth to which said microprotrusions penetrate said skin. The tip 18 preferably forms a well defined, sharp edge 20 where it meets the sides 16. The edge 20 extends substantially parallel to the abrading surface support 12 and defines a further scraping edge. In further embodiments, the edge 20 can be slightly rounded to form a smooth transition from the sides 16 to the tip 18. Preferably, the microprotrusions are frustoconical or frustopyramidal in shape.

The microabrader device 10 and the microprotrusions can be made from a plastic material that is non-reactive with the substance being administered. A non-inclusive list of suitable plastic materials include, for example, polyethylene, polypropylene, polyamides, polystyrenes, polyesters, and polycarbonates as known in the art. Alternatively, the microprotrusions can be made from a metal such as stainless steel, tungsten steel, alloys of nickel, molybdenum, chromium, cobalt, titanium, and alloys thereof, or other materials such as silicon, ceramics and glass polymers. Metal microprotrusions can be manufactured using various techniques similar to photolithographic etching of a silicon wafer or micromachining using a diamond tipped mill as known in the art. The microprotrusions can also be manufactured by photolithographic etching of a silicon wafer using standard techniques as are known in the art. They can also be manufactured in plastic via an injection molding process, as described for example in U.S. application no. 10/193,317, filed July 12, 2002 published as US 2004/0007796A1.

The length and thickness of the microprotrusions are selected based on the particular substance being administered and the thickness of the stratum corneum in the location where the device is to be applied. Preferably, the microprotrusions penetrate the stratum corneum substantially without piercing or passing through the stratum corneum. The microprotrusions can have a length up to 500 µm. Suitable microprotrusions have a length of 50 to 500 µm. Preferably, the microprotrusions have a length of 50 to 300 µm, and more preferably in the range of 150 to 250 µm, with 180 to 220 µm most preferred. The microprotrusions in the illustrated embodiment have a generally pyramidal shape and are perpendicular to the plane of the device. These shapes have particular advantages in insuring that abrasion occurs to the desired depth. In preferred embodiments, the microprotrusions are solid members. In alternative embodiments, the microprotrusions can be hollow.

As shown in Figure 2 and 5, the microprotrusions are preferably spaced apart uniformly in rows and columns to form an array for contacting the skin and penetrating the stratum corneum during abrasion. The spacing between the microprotrusions can be varied depending on the substance being administered either on the surface of the skin or within the tissue of the skin. Typically, the rows of microprotrusions are spaced to provide a density of 2 to 10 per millimeter (mm). Generally, the rows or columns are spaced apart a distance substantially equal to the spacing of the microprotrusions in the array to provide a microprotrusion density of 4 to 100 microprotrusions per mm². In another embodiment, the microprotrusions may be arranged in a circular pattern. In yet another embodiment, the microprotrusions may be arranged in a random pattern. When arranged in columns and rows, the distance between the centers of the microprotrusions is preferably at least twice the length of the microprotrusions. In one preferred embodiment, the distance between the centers of the microprotrusions is twice the length of the microprotrusions ±10 µm. Wider spacings are also included, up to 3, 4, 5 and greater multiples of the length of the micoprotrusions. In addition, as noted above, the configuration of the microprotrusions can be such, that the height to the microprotrusions can be greater than the depth into the skin those protrusions will penetrate.

The flat upper surface of the frustoconical or frustopyramidal microprotrusions is generally 10 to 100, preferably 30-70, and most preferably 35-50 µm in width.

The method of preparing a delivery site on the skin places the microabrader against the skin 28 of the patient in the desired location. The microabrader is gently pressed against the skin and then moved over or across the skin. The length of the stroke of the microabrader can vary depending on the desired size of the delivery site, defined by the delivery area desired. The dimensions of the delivery site are selected to accomplish the intended result and can vary depending on the substance, and the form of the substance, being delivered. For example, the delivery site can cover a large area for treating a rash or a skin disease. Generally, the microabrader is moved about 2 to 15 cm. In some embodiments of the invention, the microabrader is moved to produce an abraded site having a surface area of 4 cm² to 300 cm².

The microabrader is then lifted from the skin to expose the abraded area and a suitable delivery device, patch or topical formulation may be applied to the abraded area. Alternatively, the substance to be administered may be applied to the surface of the skin either before, or simultaneously with abrasion.

The extent of the abrasion of the stratum corneum is dependent on the pressure applied during movement and the number of repetitions with the microabrader. In one embodiment, the microabrader is lifted from the skin after making the first pass and placed back onto the starting position in substantially the same place and position. The microabrader is then moved a second time in the same direction and for the same distance. In another embodiment, the microabrader is moved repetitively across the same site in alternating direction without being lifted from the skin after making the first pass. Generally, two or more passes are made with the microabrader.

Methods of formulating anthrax compositions for delivery by the microneedle-mediated methods described herein are conventional. For example, a skilled worker will recognize how to use or modify in an appropriate manner methods described herein with respect to intranasal delivery.

To examine the utility of the delivery methods of the invention for the administration of biodefense vaccines, studies were carried out in animal models. The Examples below illustrate delivery with hollow and solid microneedle devices, and with intranasal administration. Examples 1-5 illustrate studies with a mouse animal model, comparing delivery with hollow and solid microneedle between the inner circumferential sidewall 4a of base 4. Alternatively, as shown in Figures 1A and 2A, handle 6 may be glued (e.g., with epoxy) to the underside 4c of base 4. Alternatively, the handle and base may be fabricated (e.g., injection-molded) together as a single two-component part. The handle may be of a diameter that is less than the diameter of the base or may be of a similar diameter as the base. Underside 4c of base 4 may be flush with mushroom-like crown 4b or extend beyond the mushroom-like crown. The lower end 6b of handle 6 may be wider than the shaft 6c of handle 6 or may be of a similar diameter as shaft. Lower end 6b may include an impression 6d that serves as a thumb rest for a person administering the substance and moving microabrader 2. In addition, protrusions 8 are formed on the outside of handle 6 to assist a user in firmly gripping handle 6 when moving the same against or across a patient's skin.

As shown in the cross-section of Figure 1B in Figure 2B, lower end 6b may be cylindrical. Microabrader 2 may be made of a transparent material, as shown in Figure 2A. Impressions 6d are disposed on both sides of the cylindrical lower end 6b to assist a person using microabrader 2 to grip the same. That is, the movement of microabrader 2 can be provided by hand or fingers. The handle 6, as well as the base 4, of the microabrader is preferably molded out of plastic or the like material. The microabrader 2 is preferably inexpensively manufactured so that the entire microabrader and abrading surface can be disposed after its use on one patient.

Abrading surface 5 is designed so that when microabrader 2 is moved across a patient's skin, the resultant abrasions penetrate the stratum corneum. Abrading surface 5 may be coated with a medicament or substance desired to be delivered to the patient's viable epidermis.

In order to achieve the desired abrasions, the microabrader 2 should be moved across a patient's skin at least once. The patient's skin may be abraded in alternating directions. The structural design of the microabrader according to the invention enables the medicament or substance to be absorbed more effectively thereby allowing less of the medicament or substance to be applied to a patient's skin or coating abrading surface 5.

Abrading surface 5 may be coated with a substance desired to be delivered to the patient. In one embodiment, the substance may be a powder disposed on abrading surface 5. In another embodiment, the substance to be delivered may be applied directly to the patient's skin prior to the application and movement of microabrader 2 on the patient's skin.

Referring to Figure 3, the microabrader device 10 of the invention includes a substantially planar body or abrading surface support 12 having a plurality of microprotrusions 14 extending from the bottom surface of the support. The support generally has a thickness sufficient to allow attachment of the surface to the base of the microabrader device thereby allowing the device to be handled easily as shown in Figure 1B, 2A and 2B. Alternatively, a differing handle or gripping device can be attached to or be integral with the top surface of the abrading surface support 12. The dimensions of the abrading surface support 12 can vary depending on the length of the microprotrusions, the number of microprotrusions in a given area and the amount of the substance to be administered to the patient. Typically, the abrading surface support 12 has a surface area of about 1 to 4 cm². In preferred embodiments, the abrading surface support 12 has a surface area of about 1 cm².

As shown in Figures 3, 4, 4A and 5, the microprotrusions 14 project from the surface of the abrading surface support 12 and are substantially perpendicular to the plane of the abrading surface support 12. The microprotrusions in the illustrated embodiment are arranged in a plurality of rows and columns and are preferably spaced apart a uniform distance. The microprotrusions 14 have a generally pyramid shape with sides 16 extending to a tip 18. The sides 16 as shown have a generally concave profile when viewed in cross-section and form a curved surface extending from the abrading surface support 12 to the tip 18. In the embodiment illustrated, the microprotrusions are formed by four sides 16 of substantially equal shape and dimension. As shown in Figures 4A and 5, each of the sides 16 of the microprotrusions 14 have opposite side edges contiguous with an adjacent side and form a scraping edge 22 extending outward from the abrading surface support 12. The scraping edges 22 define a generally triangular or trapezoidal scraping surface corresponding to the shape of the side 16. In further embodiments, the microprotrusions 14 can be formed with fewer or more sides.

The microprotrusions 14 preferably terminate at blunt tips 18. Generally, the tip 18 is substantially flat and parallel to the support 14. When the tips are flat, the total length of the microprotrusions do not penetrate the skin; thus, the length of the microprotrusions is greater than the total depth to which said microprotrusions penetrate said skin. The tip 18 preferably forms a well defined, sharp edge 20 where it meets the sides 16. The edge 20 extends substantially parallel to the abrading surface support 12 and defines a further scraping edge. In further embodiments, the edge 20 can be slightly rounded to form a smooth transition from the sides 16 to the tip 18. Preferably, the microprotrusions are frustoconical or frustopyramidal in shape.

The microabrader device 10 and the microprotrusions can be made from a plastic material that is non-reactive with the substance being administered. A non-inclusive list of suitable plastic materials include, for example, polyethylene, polypropylene, polyamides, polystyrenes, polyesters, and polycarbonates as known in the art. Alternatively, the microprotrusions can be made from a metal such as stainless steel, tungsten steel, alloys of nickel, molybdenum, chromium, cobalt, titanium, and alloys thereof, or other materials such as silicon, ceramics and glass polymers. Metal microprotrusions can be manufactured using various techniques similar to photolithographic etching of a silicon wafer or micromachining using a diamond tipped mill as known in the art. The microprotrusions can also be manufactured by photolithographic etching of a silicon wafer using standard techniques as are known in the art. They can also be manufactured in plastic via an injection molding process, as described for example in U.S. application no. 10/193,317, filed July 12, 2002, which is hereby incorporated by reference.

The length and thickness of the microprotrusions are selected based on the particular substance being administered and the thickness of the stratum corneum in the location where the device is to be applied. Preferably, the microprotrusions penetrate the stratum corneum substantially without piercing or passing through the stratum corneum. The microprotrusions can have a length up to about 500 microns. Suitable microprotrusions have a length of about 50 to 500 microns. Preferably, the microprotrusions have a length of about 50 to about 300 microns, and more preferably in the range of about 150 to 250 microns, with 180 to 220 microns most preferred. The microprotrusions in the illustrated embodiment have a generally pyramidal shape and are perpendicular to the plane of the device. These shapes have particular advantages in insuring that abrasion occurs to the desired depth. In preferred embodiments, the microprotrusions are solid members. In alternative embodiments, the microprotrusions can be hollow.

As shown in Figure 2 and 5, the microprotrusions are preferably spaced apart uniformly in rows and columns to form an array for contacting the skin and penetrating the stratum corneum during abrasion. The spacing between the microprotrusions can be varied depending on the substance being administered either on the surface of the skin or within the tissue of the skin. Typically, the rows of microprotrusions are spaced to provide a density of about 2 to about 10 per millimeter (mm). Generally, the rows or columns are spaced apart a distance substantially equal to the spacing of the microprotrusions in the array to provide a microprotrusion density of about 4 to about 100 microprotrusions per mm². In another embodiment, the microprotrusions may be arranged in a circular pattern. In yet another embodiment, the microprotrusions may be arranged in a random patters. When arranged in columns and rows, the distance between the centers of the microprotrusions is preferably at least twice the length of the microprotrusions. In one preferred embodiment, the distance between the centers of the microprotrusions is twice the length of the microprotrusions ±10 microns. Wider spacings are also included, up to 3, 4, 5 and greater multiples of the length of the micoprotrusions. In addition, as noted above, the configuration of the microprotrusions can be such, that the height to the microprotrusions can be greater than the depth into the skin those protrusions will penetrate.

The flat upper surface of the frustoconical or frustopyramidal microprotrusions is generally 10 to 100, preferably 30-70, and most preferably 35-50 microns in width.

The method of preparing a delivery site on the skin places the microabrader against the skin 28 of the patient in the desired location. The microabrader is gently pressed against the skin and then moved over or across the skin. The length of the stroke of the microabrader can vary depending on the desired size of the delivery site, defined by the delivery area desired. The dimensions of the delivery site are selected to accomplish the intended result and can vary depending on the substance, and the form of the substance, being delivered. For example, the delivery site can cover a large area for treating a rash or a skin disease. Generally, the microabrader is moved about 2 to 15 centimeters (cm). In some embodiments of the invention, the microabrader is moved to produce an abraded site having a surface area of about 4 cm² to about 300 cm².

The microabrader is then lifted from the skin to expose the abraded area and a suitable delivery device, patch or topical formulation may be applied to the abraded area. Alternatively, the substance to be administered may be applied to the surface of the skin either before, or simultaneously with abrasion.

The extent of the abrasion of the stratum corneum is dependent on the pressure applied during movement and the number of repetitions with the microabrader. In one embodiment, the microabrader is lifted from the skin after making the first pass and placed back onto the starting position in substantially the same place and position. The microabrader is then moved a second time in the same direction and for the same distance. In another embodiment, the microabrader is moved repetitively across the same site in alternating direction without being lifted from the skin after making the first pass. Generally, two or more passes are made with the microabrader.

Methods of formulating anthrax compositions for delivery by the microneedle-mediated methods described herein are conventional. For example, a skilled worker will recognize how to use or modify in an appropriate manner methods described herein with respect to intranasal delivery.

To examine the utility of the delivery methods of the invention for the administration of biodefense vaccines, studies were carried out in animal models. The Examples below illustrate delivery with hollow and solid microneedle devices, and with intranasal administration. Examples 1-5 illustrate studies with a mouse animal model, comparing delivery with hollow and solid microneedle devices to other methods of delivery. Examples 6-11 illustrate studies of lethal challenge with a rabbit model. These Example clearly show that intranasal (IN) and intradermal (ID) delivery methods provide superior protection compared to Conventional delivery methods, such as intramascular (IM) injection. Furthermore, the abrader/topical administration method of the invention provides superior protection compared to convention methods of topical delivery.

In the foregoing and in the following examples, all temperatures are set forth uncorrected in degrees Celsius; and, unless otherwise indicated, all parts and percentages are by weight

### EXAMPLES

### I. Studies in a mouse model

### Example 1 - Experimental design for studies of the administration of rPA with hollow and solid microneedle devices

**Formulations:** Liquid formulations only
**Adjuvants:** see Experimental Groups below
**Immunization Schedule:** Mice were immunized at d0, d21 and d42
**Antigen Dose:** 10µg rPA per mouse per immunization
**Number of animals:** Total =10/group
**Sample Collection:** Blood was collected on day 0 ("d0", pre-bleed), d21 (prior to immunization 2), d42 (prior to immunization 3), and d56. Bronchialalveolar lavage (BAL) fluid was collected from at least 5 animals per group at d56.
**Challenge: None**
**Assays:** Serum from individual mice was analyzed for rPA-specific total Ig by ELISA, using standard methods of the art. In addition, pooled group sera was analyzed for rPA-specific Ig isotype profiles (IgG1, IgG2aIgG2b, IgG3, IgM, IgA) using a sub-isotyping kit assay (Quantitative ELISA Immunoassay, Bethyl Laboratories, Montgomery, TX). BAL fluid from individual mice was analyzed for rPA-specific total Ig and IgA. Anthrax neutralization was measured by adding dilutions of sera from vaccinated or control animals to monocyte cell cultures incubated with lytic concentrations of PA and lethal toxin (LT) from *B. anthracis.* This assay represents an *in vitro* correlate of protection against anthrax disease. Cell viability was determined by measuring intracellular ATP.

### Experimental Groups:

Group 1: IM - alum*
Group 2: IN (liquid instillation) - No adjuvant
Group 3: IN (liquid instillation) - CpG**
Group 4: ID (HMN) - No adjuvant
Group 5 : ID (HMN) - alum
Group 5: ID (HMN) - alum
Group 6: ID (HMN) - CpG
Group 7: SMN (protocol 1 = "pre-abrasion") - No adjuvant
Group 8: SMN (protocol 1 = "pre-abrasion" - alum
Group 9: SMN (protocol 1= "pre-abrasion") - CpG
Group 10: SMN (protocol 2 = "abrasion thru droplet") - No adjuvant
Group 11: SMN (protocol 2 = "abrasion thru droplet") - alum
Group 12: SMN (protocol 2 = "abrasion thru droplet") - CpG
Group 13: Topical - No adjuvant.
Group 14: Topical - alum
Group 15: Topical - CpG

* Alhydrogel (2% AL(OH)3; Superfos Biosector)
** CpG oligonucleotide (TCCATGACGTTCCTGACGTT; Proligo, LLC), 10 µg/mouse

A recently developed candidate anthrax vaccine is based on a recombinantly produced Protective Antigen (rPA) (Ivins, B.E., Infection and Immunity, 60:662-668, 1992; Ramirez, D.M., et al., J. Industrial Microbiol and Biotechnol. 28:232-238, 2002). This vaccine is being investigated by USAMRIID as an alternative to AVA due to the potential for improved efficacy and thus a reduction in the required number of booster administrations, as well as an improved safety profile. Additional pre-clinical and clinical studies are required in order to determine the most effective delivery routes for protection and to further elucidate the potential requirements for adjuvants. This vaccine was used in the examples set forth below.

For HMN studies, a hollow 1 mm 0.16 mm (34 gauge) stainless steel needle was attached to a short section of catheter tubing, which was in turn attached to a syringe. Injections into the mice were inserted essentially parallel to the skin surface in a Mantoux-style injection.

For SMN studies, the device comprised a plastic abrading surface on a hand held applicator, as shown in Figures 1-5 with an abrading surface consisting of 200 µm tall plastic frustoconical microprojections, as shown in Figure 4. The abrading surface was fitted onto a microabrader device, as depicted in Figures 1 and 2. Technology to produce these and similar abraders is known to those of skill in the art.

At each application site, a total of six passes was made over the shaved skin of the mouse so that an area of approximately 1.5 cm² was abraded. For some SMN experiments, the skin was abraded and the vaccine applied ("pre"), for others the vaccine was applied and the skin immediately abraded through the droplet ("abr").

A volume of 50 µl was split between two injection or abrasion sites for all experiments, unless indicated otherwise.

IM injection was performed in a standard manner at one site using a standard 0.25 mm (30 gauge) needle. Intranasal administration was performed by liquid instillation of 30 µl (15 µl per each nostril) using a conventional Gilson pipettor (Gilson, Middleton, WI) For topical administration, the vaccine was applied to shaved skin.

### Example 2 - PA-specific Serum Antibody Response (Total Ig)

Table 1 below displays the anthrax rPA-specific serum antibody titers (1/serum dilution) for individual mice (n=10 per group) at d21 post-immunization; i.e., following a single dose of vaccine. Sero-positive responses are indicated in bold.

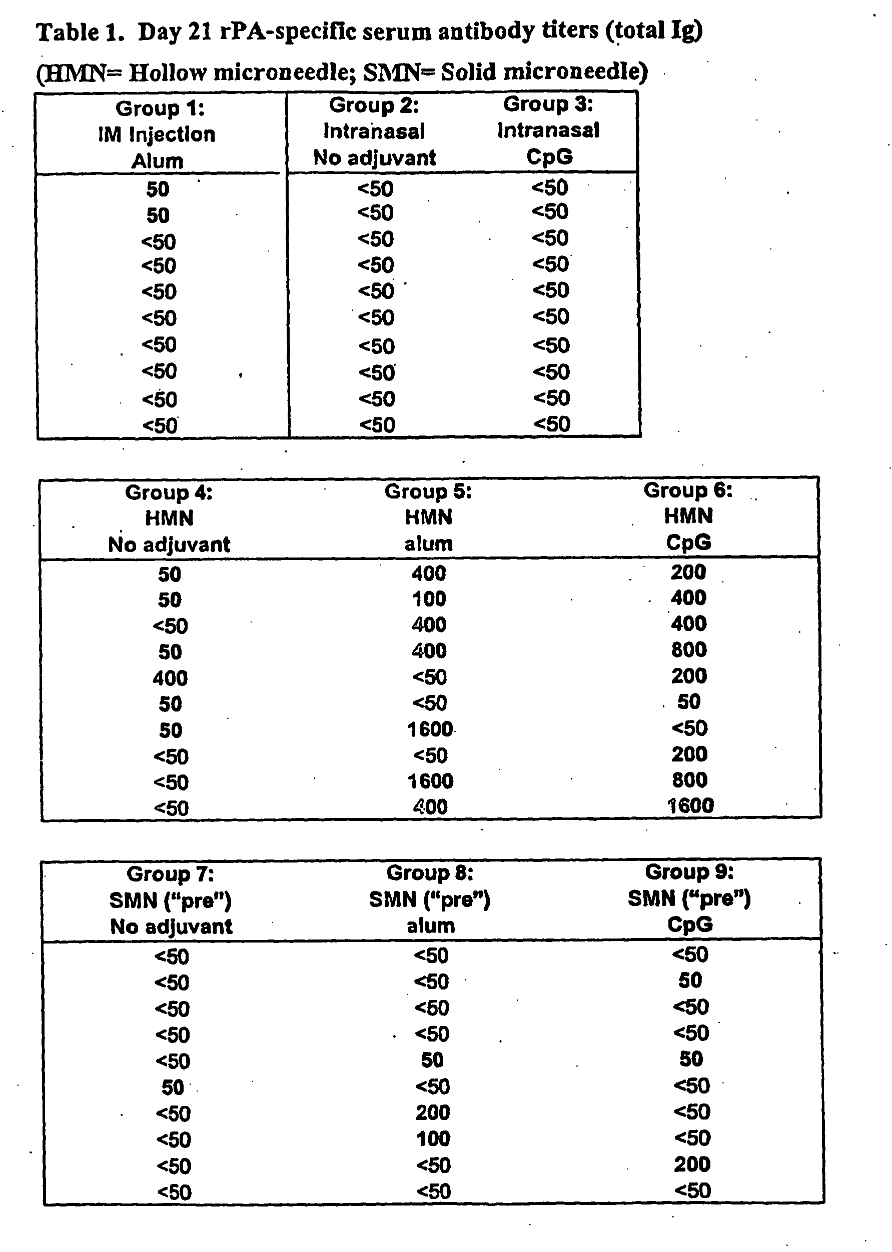

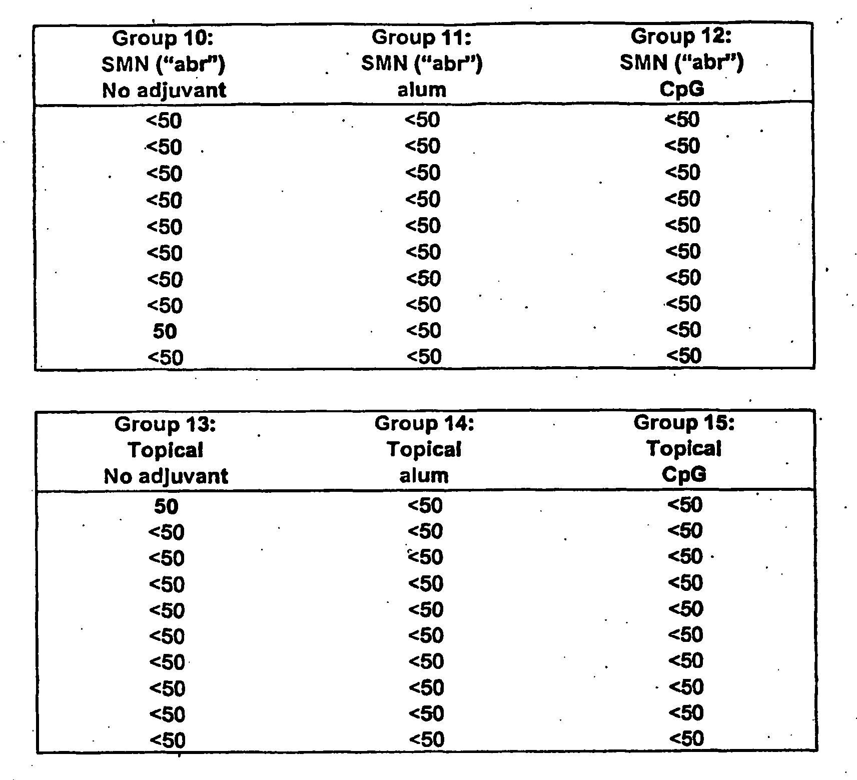

The results indicate that the strongest serum antibody titers and highest overall response rates were observed following HMN-based ID delivery. Current anthrax vaccines contain alum as adjuvant and are administered either IM or SC. There was only a 20% response rate in the IM-alum group (Group 1), as compared to a 60% response rate in animals immunized ID in the absence of adjuvant (Group 4). The response rates in the ID groups were further enhanced by the addition of either alum (80%; Group 5) or CpG (90%; Group 6) as adjuvant.

SMN-based delivery stimulated an antibody response of similar magnitude and response rate (up to 30%; Groups 8 and 9) as that induced by the conventional IM-alum regimen. In addition, the SMN devices enabled topical delivery, as only a single positive responder was evident across all 30 mice receiving rPA topically without a micro-abrasion device (Groups 13-15).

IN delivery did not result in a positive antibody response after a single dose of vaccine.

Altogether, the results indicate that, relative to the conventional IM-alum vaccine regimen, HMN-based ID delivery of anthrax rPA overcomes the need for adjuvants, and increases the serum antibody response level and response rate. In addition, the results indicate that SMN-based topical administration to the skin induces an antibody response that is at least as strong as that induced by the conventional IM-alum regimen.

Tables 2 and 3, below, illustrate the anthrax rPA-specific serum antibody titers (1/serum dilution) following 2 and 3 doses of vaccine, respectively. Anesthesia-related deaths not related to any particular method of delivery or adjuvant occurred in some animals and are indicated in the Tables below.

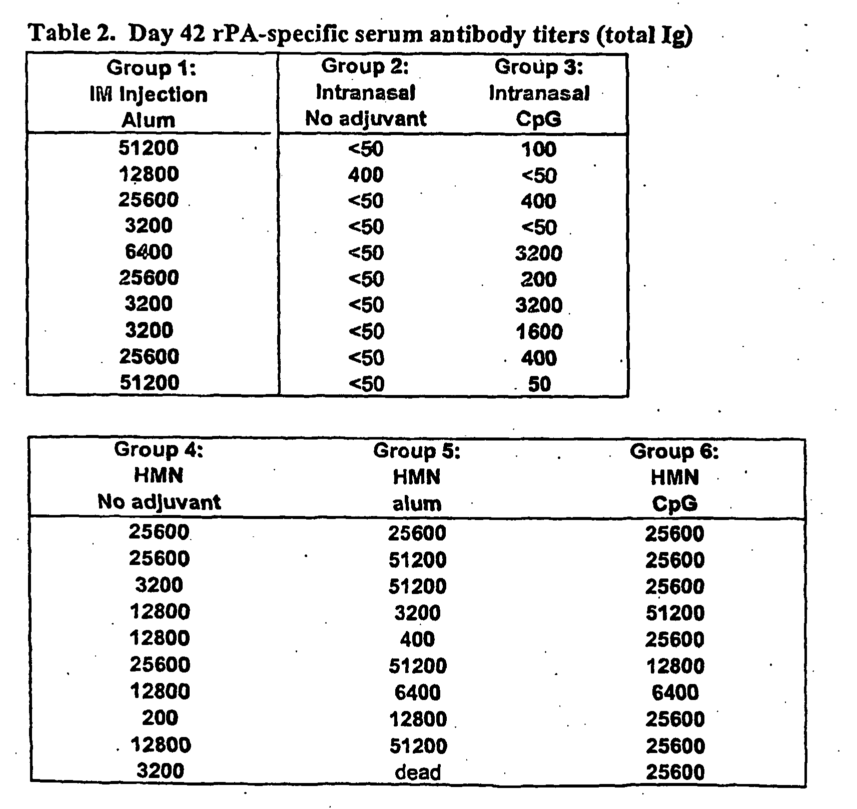

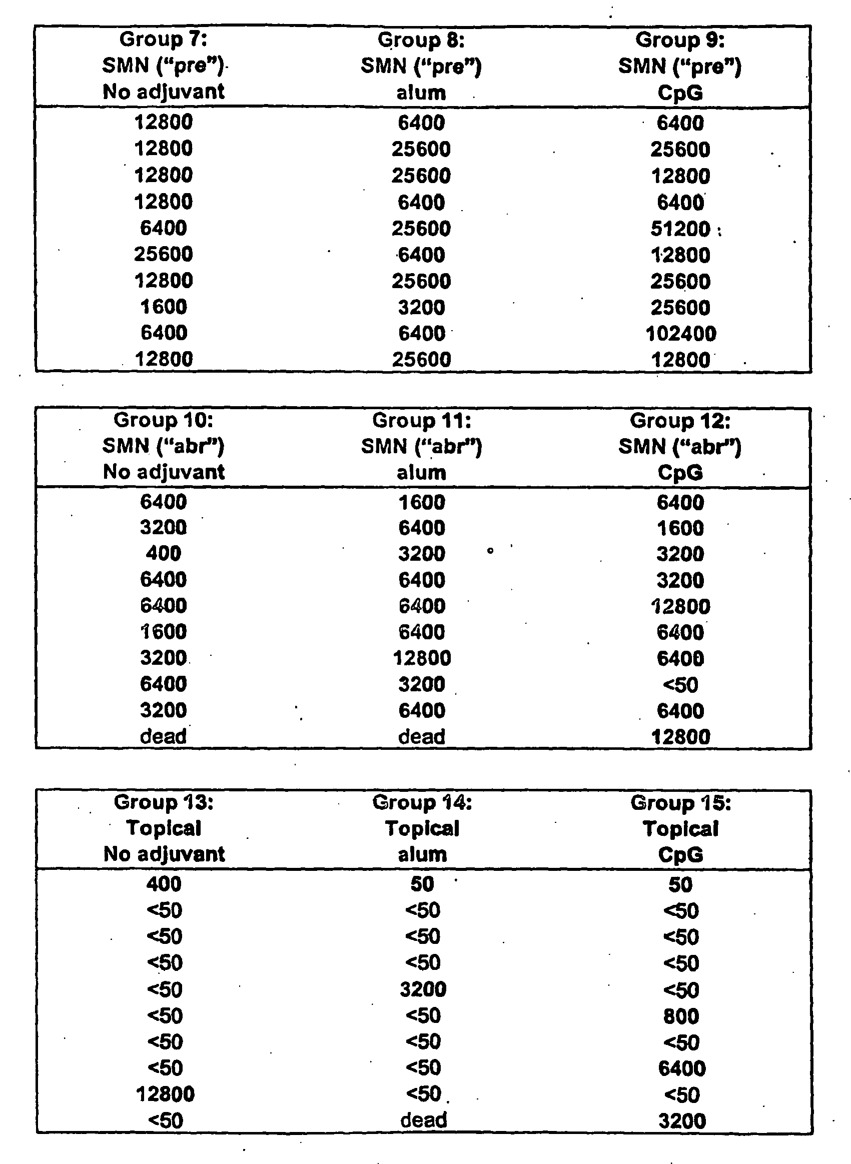

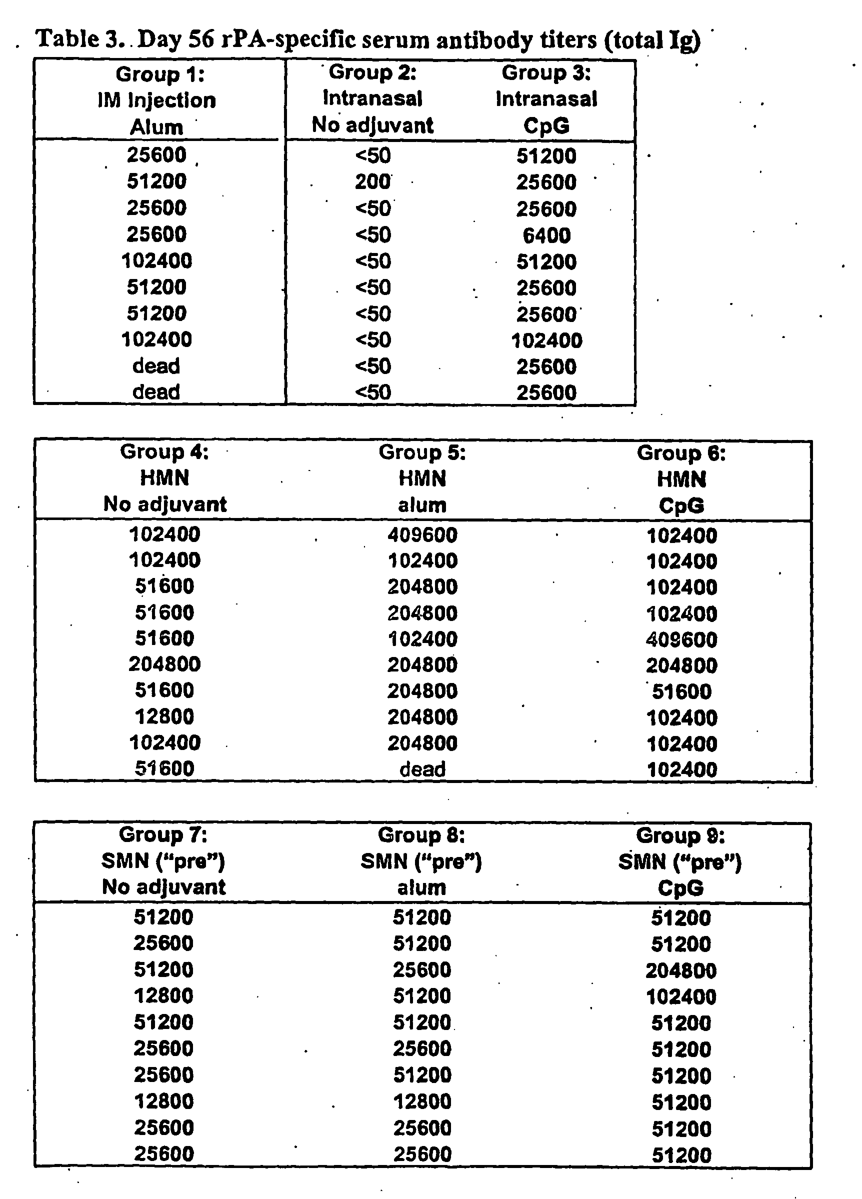

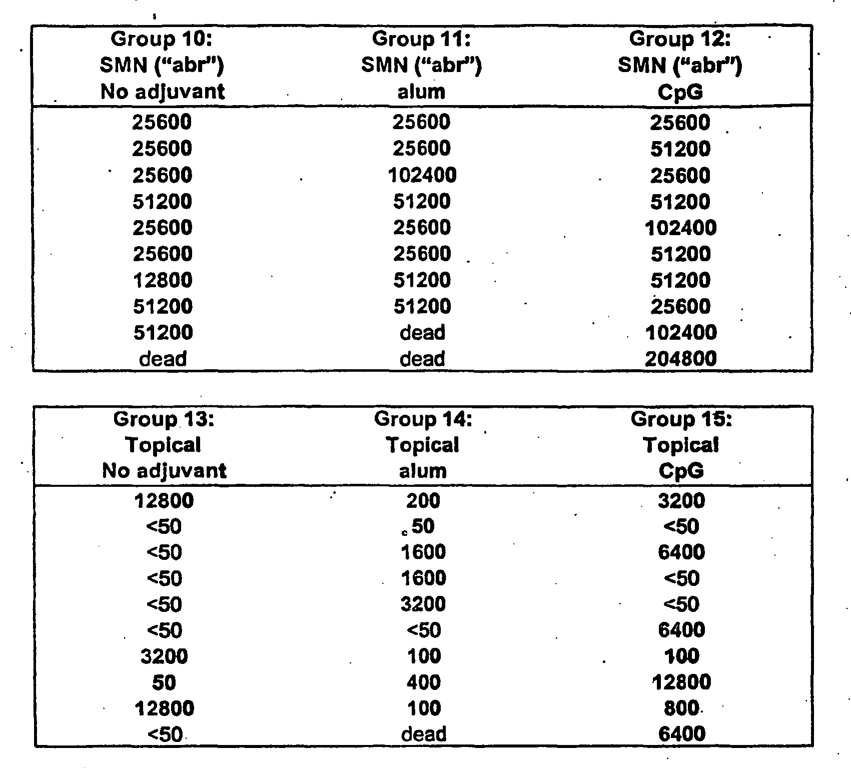

The results presented in Tables 2 and 3 demonstrate that there is a significant booster effect upon administration of the 2^{nd} and 3^{rd} dose of rPA vaccine in all groups. At the completion of the study (d56), the strongest overall mean serum antibody titers were observed in the ID-HMN groups (Group 4 mean titer = 78,280; Group 5 mean titer = 204,800; Group 6 mean titer = 138,280). In all cases the mean ID-induced titers were stronger than the corresponding titer achieved via the conventional regimen of IM injection of rPA plus alum (Group 1 mean titer = 54,400). Thus, as indicated above, HMN-based ID delivery induces a stronger serum antibody response than via the conventional regimen of IM-alum and reduces or eliminates the need for adjuvants.

In addition, the results indicate that SMN induces antibody responses that are of comparable or greater magnitude than those induced via the conventional regimen of IM-alum. At d56, mean SMN-induced titers ranged from 30,720

(Group 7) to 71,680 (Group 9), as compared to a mean titer of 54,400 in the IM-alum group (Group 1). Thus, SMN delivery also reduces or eliminates the need for adjuvants and, in the presence of adjuvants, induces an immune response that is at least as strong as that induced by IM injection, yet without a conventional needle-based injection.

In addition, the results indicate that IN delivery of anthrax rPA requires use of an adjuvant, in this case CpG, to induce a significant immune response and 100% sero-conversion. Upon addition of adjuvant, however, an antibody response of similar but slightly lower magnitude than via IM injection is achieved (Group 3 mean titer at d56 = 36,480), yet without a conventional needle-based injection.

### Example 3 - PA-specific Serum Antibody Response (Ig Isotopes)

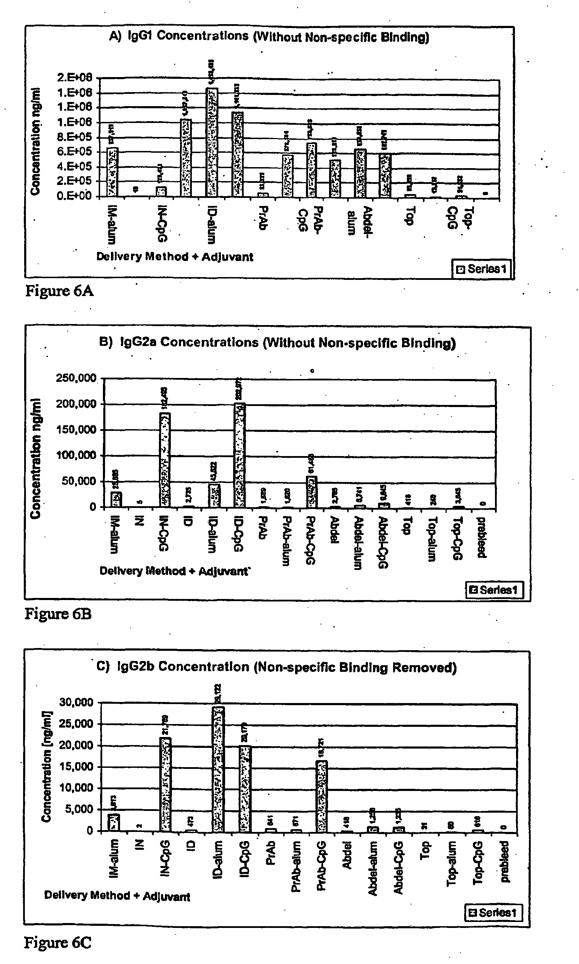

To further characterize the immune response, d56 sera from the experimental groups presented in Example 1 above were further investigated for Ig isotype profile. Different isotypes are involved in different immune functions; e.g., IgG fixes complement and binds to Fc-receptors, IgE is involved in allergic responses, IgA is involved in mucosal immune responses. Helper T (Th) cells can be divided into 2 subsets, based on the particular cytokines produced; "Th1" cells produce the pro-inflammatory cytokines IFN-γ and TNF-α important in cellular immune responses, whereas "Th2" cells produce IL-4, IL-5 and IL-10 which are important for humoral immune responses. Both Th1 and Th2 cytokines influence IgG isotype switching; "Th1" cytokines favor production of IgG2a, whereas "Th2" cytokines favor production of IgG1. Thus, the ratio of IgG1:IgG2a is suggestive of the type of helper T cell response induced by a given vaccine. Figures 6A-6F show the d56 serum concentrations of rPA-specific antibodies of the various isotypes in the experimental groups presented in Example 1.

The results presented in Figure 6 indicate that the majority of the antibodies produced in all groups consisted of IgG1, IgG2a and IgG2b. Very little or no detectable antibody of the IgM, IgA and IgE isotypes were present in the serum. Thus, the novel methods and devices described herein induce an antibody response that is similar in composition to that achieved via conventional IM injection. Notably, the novel methods and devices described herein did not favor induction of an allergic IgE type response.

Table 4, below, displays the IgG1:IgG2a ratios for the experimental groups presented in Example 1. In groups receiving rPA either without adjuvant or with alum as adjuvant, IgG responses displayed a higher IgG1:IgG2a ratio than the corresponding groups immunized with rPA plus CpG. Altogether, the novel methods and devices described herein appeared to induce IgG1:IgG2a ratios as expected for these types of adjuvants. However, the IgG1:IgG2a ratios in the IN groups were, in general, lower than those present in other groups, suggesting more Th1 activity following IN delivery than that achieved via the other routes.

**Table 4. IgG1 :IgG2a ratios (refer to Example 1 for description of Experimental Groups)**

| | | | | | | | | | | | | | | | |
|---|---|---|---|---|---|---|---|---|---|---|---|---|---|---|---|
| **Group:** | **1** | **2** | **3** | **4** | **5** | **6** | **7** | **8** | **9** | **10** | **11** | **12** | **13** | **14** | **15** |
| **G1/G2a Ratio:** | **23** | **4** | **0.7** | **379** | **32** | **6** | **33** | **356** | **12** | **182** | **114** | **61** | **84** | **56** | **9** |

### Example 4 - PA-specific Antibody Response in BAL (Total Ig)

Anthrax-specific antibodies present in the BAL provide a first line of defense against the inhalation form of the disease. At the termination of the experiment described in Example 1, BAL samples were collected from 5-10 mice per group and analyzed for PA-specific antibodies by ELISA. PA-specific antibody titers (1/BAL dilution) are presented in Table 5, below:

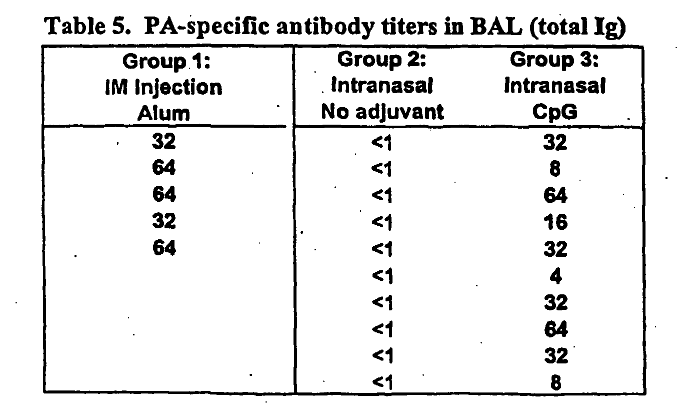

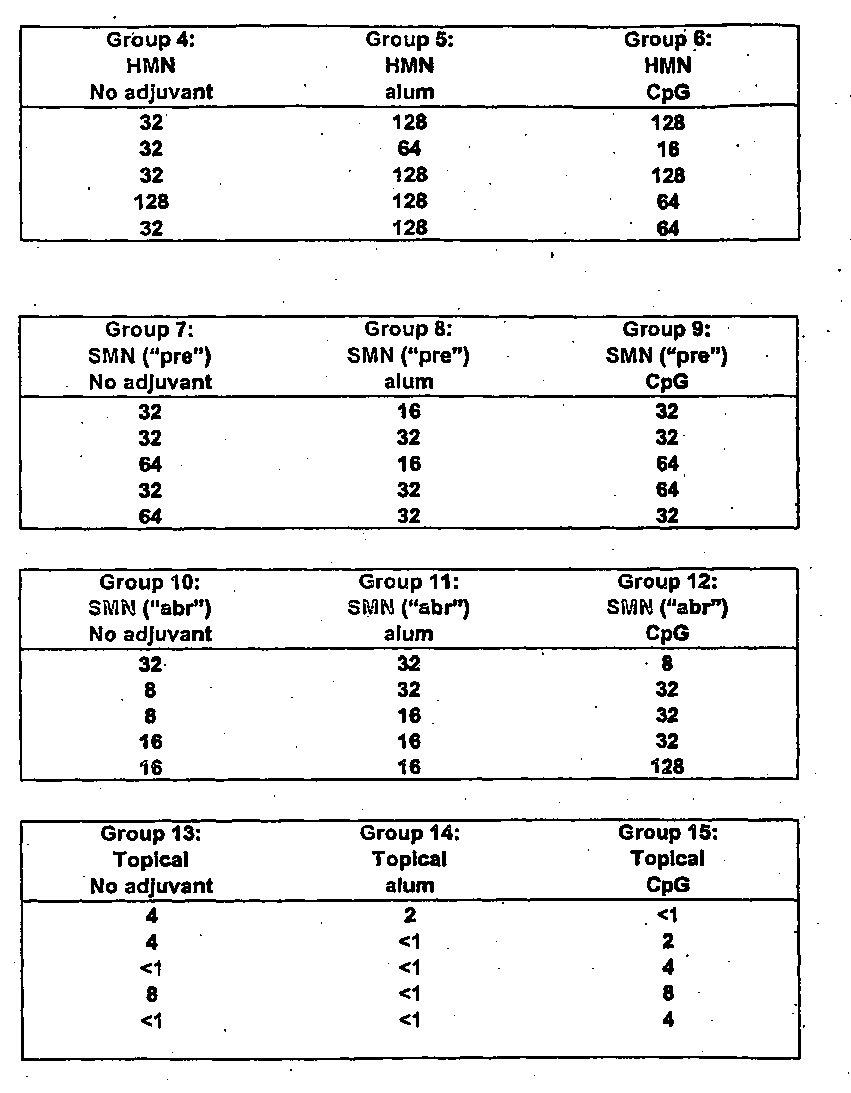

The results presented in Table 5 indicate that HMN- and SMN-based delivery of anthrax rPA vaccine induce significant levels of PA-specific antibodies in the BAL fluid. As with the serum titers, HMN-based delivery resulted in the strongest overall titers in the BAL with individual mice responding with a mean group titer as high as 115 (Group 5), as compared to a mean group titer of 51 following IM injection (Group 1). Notably, animals immunized via HMN raised a significant titer in the BAL even in the absence of added adjuvant. Likewsie, SMN-based delivery resulted in the induction of significant titers in the BAL fluid even in the absence of added adjuvant. In addition, SMN-induced titers were comparable in magnitude to those generated by the conventional IM-alum regimen. Thus, the methods and devices of the present invention are able to overcome the requirements for adjuvants in the induction of immune responses in the BAL fluid. Such immune responses in the BAL are likely to be important for protection against aerosol challenge with anthrax spores.

IN delivery required an adjuvant in order to induce significant antibody titers in the BAL fluid. In the presence of adjuvant, however, rPA administered IN yielded similar but slightly lower BAL titers as compared to IM injection.

In addition to the total Ig analysis presented above, BAL fluid was also analyzed for PA-specific IgA. IgA is a secretory antibody involved in protection from infection at mucosal surfaces. PA-specific IgA was only detectable in a limited number of samples; 4/9 samples from the IN/CpG group (Group 3) and 1/5 samples from the M/CpG group (Group 6) were positive at dilutions ranging from 1:2 to 1:8. All other samples were negative. These results indicate that IN and ID delivery are unique among the routes tested in their ability to induce IgA responses in the BAL fluid.

### Example 5 - Serum Neutralizing Antibody Titers

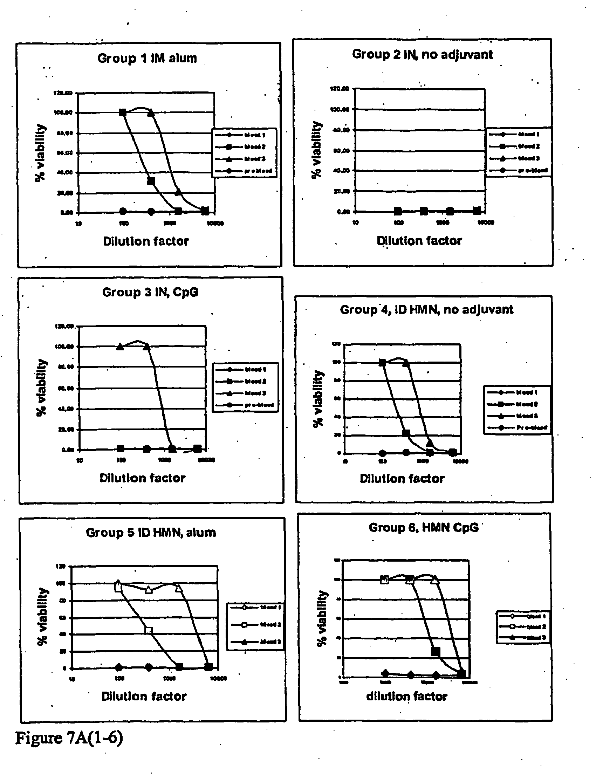

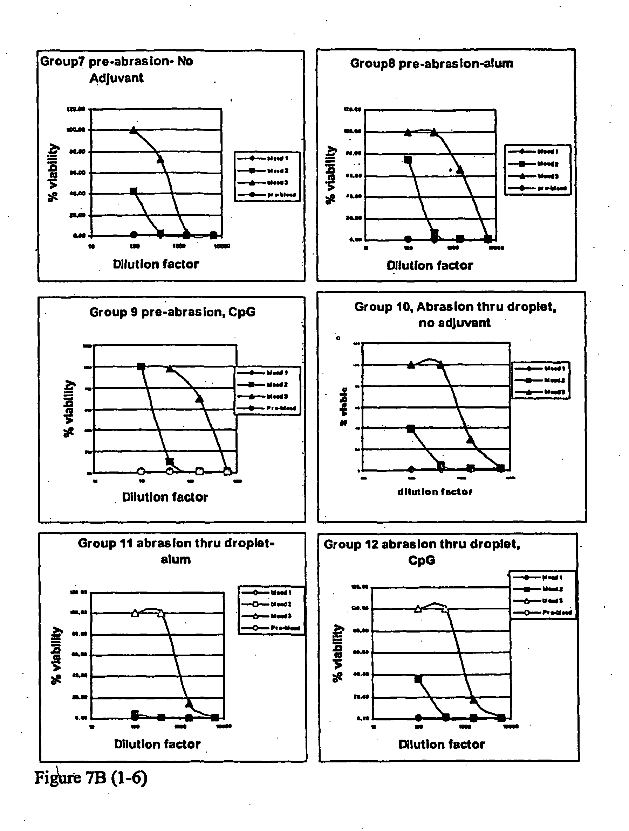

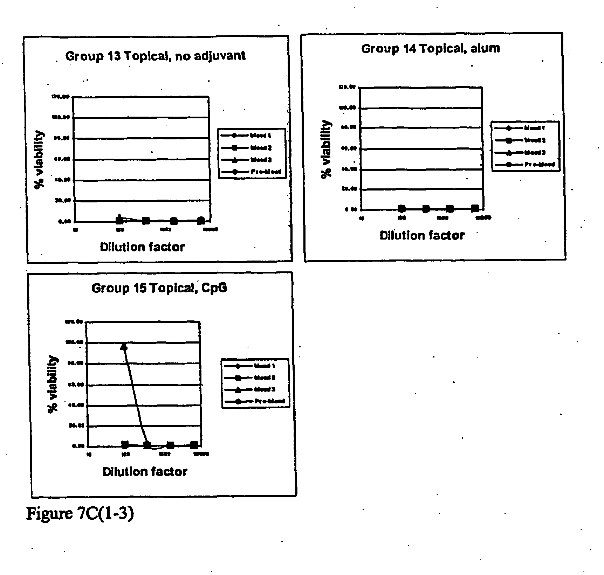

Anthrax neutralization was measured by adding dilutions of sera from vaccinated or control animals to monocyte cell cultures incubated with lytic concentrations of PA and lethal toxin (LT) from *B. anthracis.* This assay represents an *in vitro* correlate of protection against anthrax disease. Cell viability was determined by measuring intracellular ATP. Results are presented in Figure 7. Notably, HMN-based ID delivery of rPA without added adjuvant induced similar serum Nab titers as IM plus alum. Addition of alum or CpG as adjuvant further increased the Nab titer relative to IM. Similarly, SMN-based delivery also induced significant serum Nab titers that, in the presence of adjuvant, were greater than those induced by IM injection. Pre-treatment with the SMN microabrader device followed by topical application of the vaccine resulted in slightly stronger responses than those achieved via the alternate protocol of abrading directly through the vaccine droplet on the skin surface: IN delivery failed to induce Nabs in the absence of adjuvant; but did induce Nabs after 3 doses of rPA plus CpG. In the BAL, low Nab titers were observed in the SMN and HMN groups, whereas IM and IN treated animals were negative (data not shown).

Thus, ID delivery via HMN devices induces stronger anthrax neutralizing antibody titers than any other route of delivery. SMN enables topical delivery and induces anthrax neutralizing antibody titers that are as strong or stronger than those achieved via the IM injection. Notably, neutralizing antibodies were present in the HMN and SMN groups even in the absence of added adjuvant. Thus, the methods and devices of the present invention overcome the need for added adjuvants in the induction of a protective immune response to a vaccine. IN delivery also induced neutralizing antibody responses but required an adjuvant. Thus, IN delivery yields comparable results to IM injection but does so via non-invasive delivery without a needle.

### II. Lethal challenge studies in a rabbit model

### Example 6 - Experimental design for studies comparing the efficacy of various delivery routes and other factors

### Delivery routes:

### Hollow microneedle (HMN)-based intradermal (ID) delivery:

Injections into rabbits were made perpendicular to the skin surface, using a 34 gauge needle, with a 1 mm depth, on the end of a catheter line. The delivery rate was not controlled. The administration was performed by hand, over an about 20-30 second time span, using a rapid bolus injection. One hundred microliters of vaccine was administered, divided over 2 delivery sites per rabbit.
**Solid microneedle (SMN) abrasion-based delivery:** Rabbits were shaved with electric clippers then depilated using Nair® cream. Rabbits were then pre-treated with the SMN device, as described above. Vaccine (100 µl volume divided over 2 treatment sites, each of approximately 1x3cm area) was then topically applied to the treated skin using a Gilson pipetter.
Topical delivery (no device): Rabbits were shaved with electric clippers then depilated using Nair® cream. Vaccine (100 µl volume divided over 2 treatment sites, each of approximately 1x3cm area) was then topically applied to the treated skin using a Gilson pipetter.
**Intramuscular (IM) injection:** Vaccine (100 µl volume) was injected into the quadriceps muscle using a standard 30Ga needle and 1cc syringe.
**Intranasal (IN) (liquid formulation):** Vaccine (100 µl volume) was sprayed into one nostril using a Penn-Century nasal sprayer device.
**Intranasal (IN) delivery, with an aerodynamically light particle (ALP) formulation of the invention:** Each capsule was filled with 10 mg of powder. Delivery was accomplished by pushing 5 ml of air through the device and into the nostril. Adjuvants: Alhydrogel (2% AL(OH)3; Superfos Biosector), CpG oligonucleotide (TCCATGACGTTCCTGACGTT; Proligo, LLC), 50 µg/rabbit.
Animals: New Zealand White (NZW) female rabbits
**Immunization schedule:** Rabbits were immunized at d0, d21 and d42
Antigen dose: 50µg rPA per immunization
**Number of animals:** Total = n=6 or n=9 per group. Six animals from each group were subjected to lethal challenge. The remaining animals were sacrificed for broncheal alveolar lavage (BAL), nasal, or vaginal lavage on day 56. See Experimental Groups below
**Sample Collection:** Blood was collected on day 0 ("d0", pre-bleed). d21 (prior to immunization 2), d35 (prior to immunization 3), and d56.
**Assays:** Assays were conventional and/or were performed as described elsewhere herein.

Serum total Ig titers were measured on day 21, 35 and 56. Serum from individual rabbits was analyzed for rPA-specific total rPA-specific total Ig by ELISA. BAL total Ig titers were measured on day 56. BAL IgA titers were measured on day 56. Nasal IgA titers were measured on day 56, Vaginal IgA titers were measured on day 56. Serum IgA titers were measured on day 56. PA/LT-neutralizing titers were determined in monocyte cell cultures on day 21, 35 and 56, as described above..

Lethal aerosol challenge was conducted with ~100 LD₅₀ anthrax spores, as described previously (Pitt et al., Vaccine 19:4768-4773, 2001). Challenge was performed 6 weeks following the 3^{rd} dose of vaccine.

**Table 6: Experimental Groups:**

| **Group #** | **n** | **Administration** | **Adjuvant** |
|---|---|---|---|
| 1 | *9 | IN | Liq, CpG |
| 2 | *9 | IN Solovent | FD, CpG |
| 3 | *9 | IN Solovent | SFD, CpG |
| 4 | *9 | IN Solovent | FD, Chit + CpG |
| 5 | *9 | IN Solovent | SFD, Chit + CpG |
| 6 | *9 | ID HMN | CpG |
| 12 | 6 | ID HMN | |
| 13 | 6 | ID HMN | Alum |
| 9 | 6 | IM | CpG |
| 10 | 6 | IM | Alum |
| 11 | *9 | IM | |
| 7 | *9 | SMN | CpG |
| 14 | 6 | SMN | Alum |
| 8 | 6 | Topical | CpG |

| | | | |
|---|---|---|---|
| * Indicates groups with animals sacrificed for BAL, Nasal and Vaginal lavage. | | | |

### Preparation of SFD rPA particles:

For the dry powder SFD rPA samples used in this example, liquid particles were generated from solutions containing rPA and excipients using a BD AccuSpray nozzle as described in U.S. application no. 10/299,012 published as US 2003/0180755A1. The volume mean diameter of liquid particles produced by this process is between 50µm and 100µm. The frozen droplets were dried by lyophilization (freeze-drying, under vacuum), using a conventional lyophilization apparatus.

The compositions used in the rabbit challenge study were:

| | **Group 3** | **Group 5** |
|---|---|---|
| rPA | 1.231 ml (1.125 mg) | 1.231 ml (1.125 mg) |
| Trehalose | 0.2246 g | 0.2041 g |
| CpG | 0.512 ml (1.1264 mg) | 0.512 ml (1.1264 mg) |
| Chitosan | --------- | 0.0064 g |

In the following examples, particles were frozen by spraying them into a vessel (such as a Virtis freeze-drying flask) containing liquid nitrogen, the vessel was then attached to a conventional lyophilizer and the excess liquid nitrogen evaporated off. The frozen aerosol was dried within about 48 to reach a moisture level below 3 wt%.

For sample 4, chitosan was spray freeze dried from a solution containing 20 wt% chitosan and 80 wt% trehalose, using a BD AccuSpray nozzle as described above. The SFD rPA and SFD chitosan were then blended by agitation in a vial.

### Preparation of FD rPA particles:

For the dry powderFD rPA samples used in this example, the frozen solution was dried by lyophilization (freeze-drying, under vacuum), using a conventional lyophilization apparatus. This method is generally called a " freeze-dry" or FD method, and compositions made by the method are called "freeze-dry" or FD compositions.

The compositions used in the rabbit challenge study were:

| | **Group 2** | **Group 4** |
|---|---|---|
| rPA | 1.477 ml (1.35 mg) | 1.477 ml (1.35 mg) |
| Trehalose | 0.2699 g | 0.2402 g |
| CpG | 0.614 ml (1.3508 mg) | 0.614 ml (1.3508 mg) |
| Chitosan | --------- | 0.0075 g |

Solutions containing rPA, trehalose, and CpG were frozen by placing vials containing the solutions into a dry ice bath. The vials containing frozen solution were then dried using a conventional lyophilization apparatus to reach a moisture level below 3 wt%. For sample 3, the dried powder was then milled for 30 using a "wiggle bug" ball mill. Sample 5 was prepared by adding to the dried sample SFD chitosan, which was prepared as described above, and milling for 30 using a "wiggle bug" ball mill. This milling process results in samples with a volume mean diameter of between 50µm and 100µm.

### Example 7 - Serum antibody titers on day 21, day 35 and day 56

Serum antibody titers on day 21, day 35 and day 56 are presented in Tables 7-9.

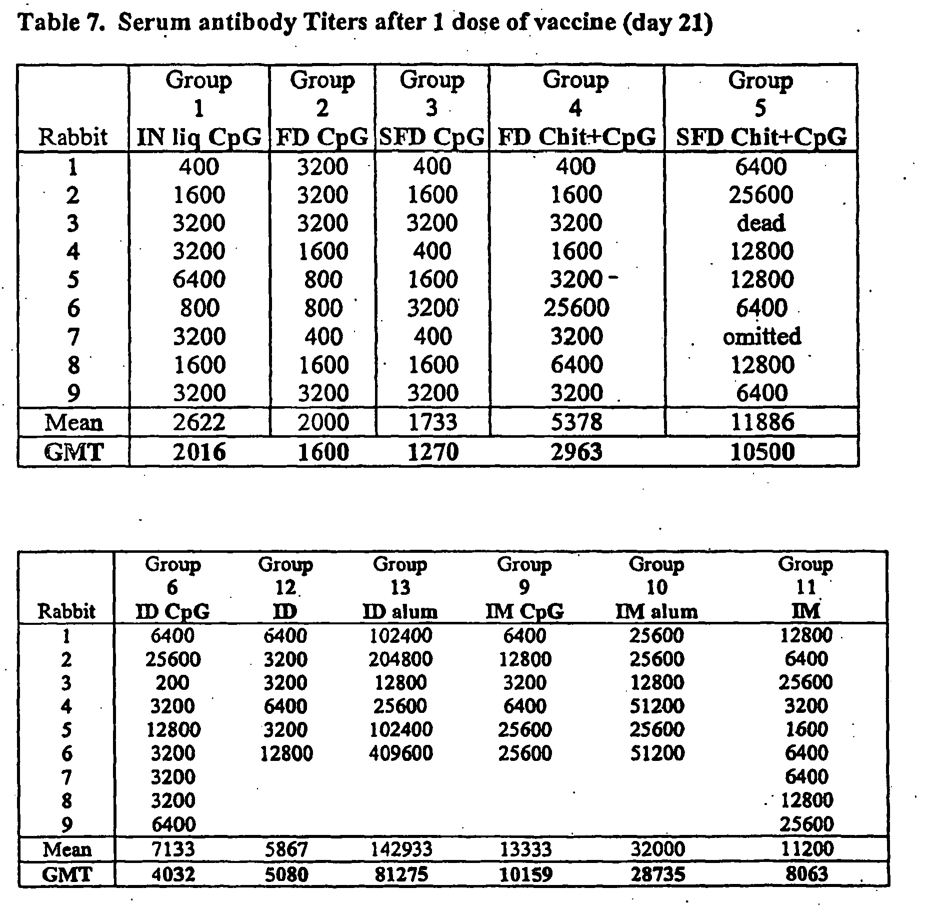

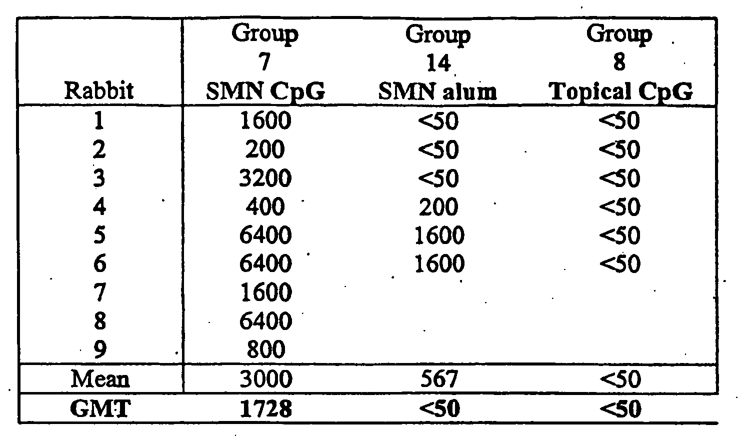

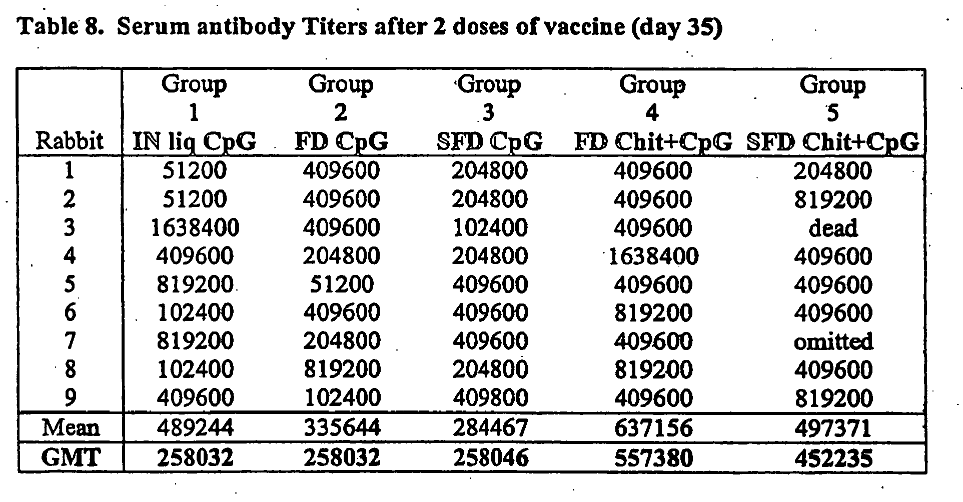

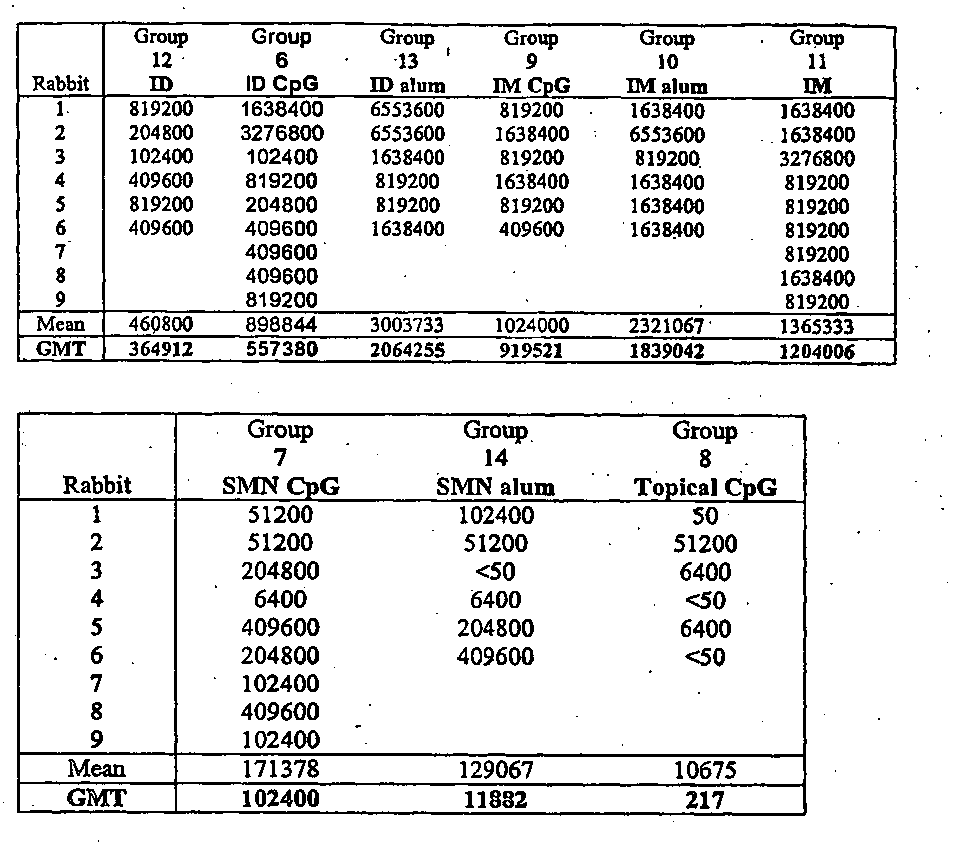

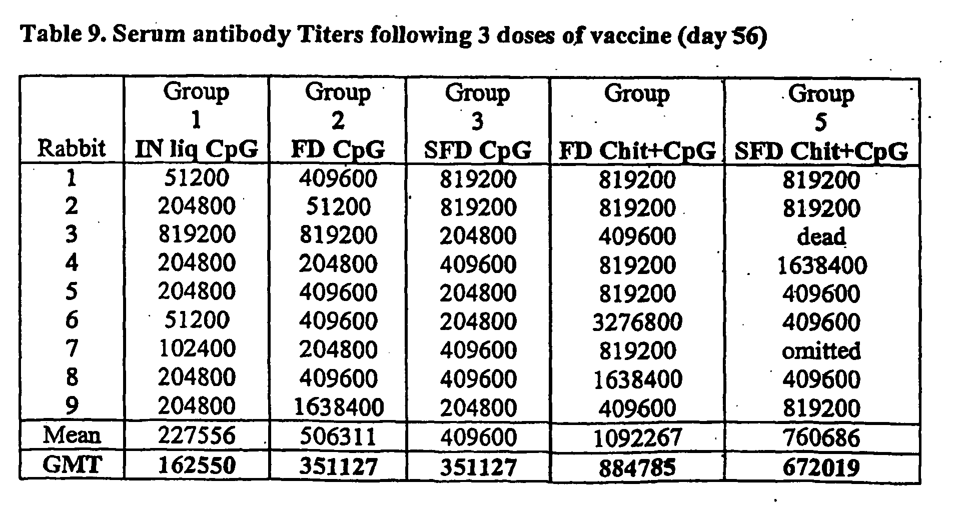

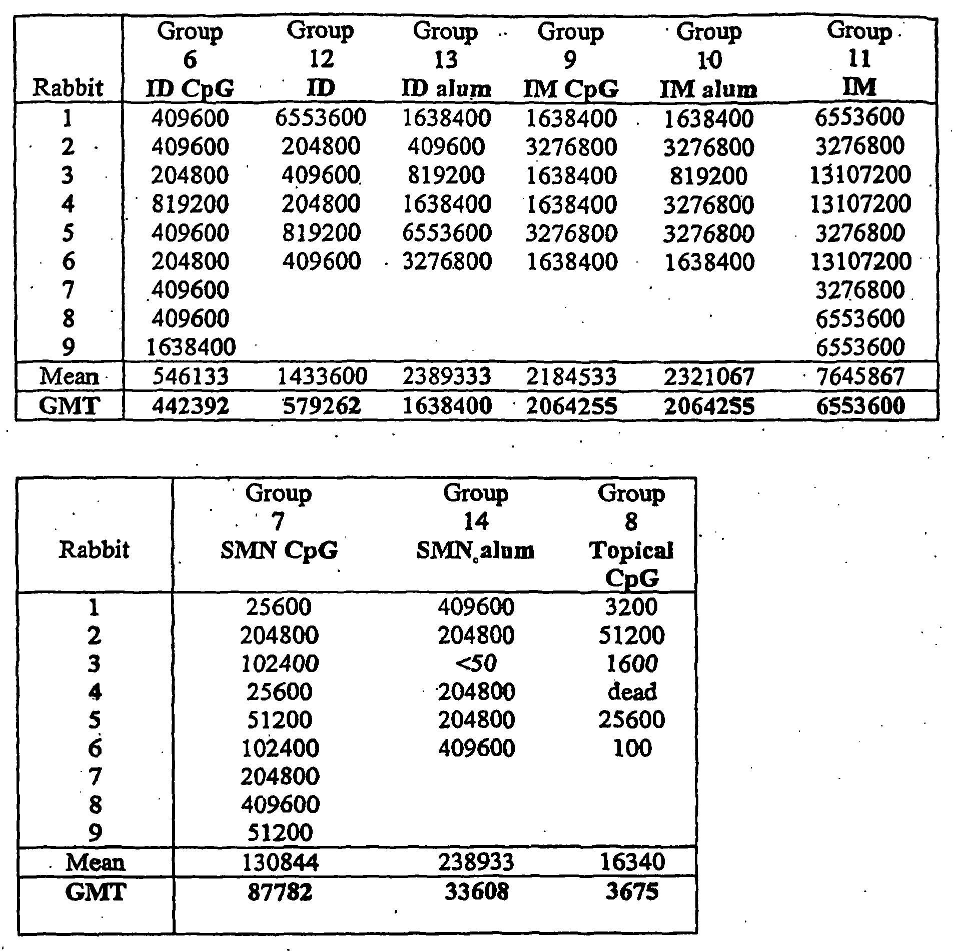

After 1 dose of vaccine, the highest overall serum antibody response was in the ID-alum group, consistent with the results of the mouse study presented above. The arithmetic mean titer in this group was 142,933 as compared to a mean titer of 32,000 in the IM-alum group. IN delivery also induced significant serum antibody titers after a single dose of vaccine, although generally lower than those achieved by ID or IM injection. Rabbits immunized IN with the SFD powder formulation containing chitosan and CpG adjuvant generated the strongest overall IN-induced response with a mean serum titer of 10,500 as compared to a titer of 2016 in the group immunized IN with the standard liquid formulation.

SMN devices enabled topical delivery of anthrax vaccine, as significant antibody responses were generated after 1 dose of vaccine in groups 7 and 14, whereas none of the rabbits in the topical group (group 8) generated a detectable response.

After the 2^{nd} dose of vaccine, all groups showed a significant rise in serum antibody titers. As after the first dose, the strongest overall response was generated in the HMN ID-alum group with a mean titer of 3,003,733 as compared to a mean of 2,321,067 in rabbits immunized IM with rPA plus alum.

After the 3^{rd} dose of vaccine, all groups were positive for PA-specific antibodies in the Serum. Overall, the ID and IM induced titers were highest, followed by IN, SMN and topical.

### Example 9 - Mucosal Antibody titers on day 56

Tables 10-12 show antibody titers on day 56 in various tissues. Table 10 shows PA-specific IgA titers in BAL fluid; Table 11 shows PA-specific IgA titers in nasal fluid; Table 12 shows PA-specific IgA titers in vaginal fluid. The results presented in Table 10 show that the anthrax vaccine compositions and methods of the present invention induce mucosal immune responses in the BAL. The results presented in Table 11 show that the anthrax vaccine composition and methods of the present invention induce mucosal immune responses in the nasal cavity. The results presented in Table 12 show that the anthrax vaccine composition and methods of the present invention induce mucosal immune responses in the vaginal tissue, a remote mucosal tissue distant from the sites of vaccine administration.

**Table 10 Antibody Titers (total IgA) in BAL fluid (day 56)**

| | **Group 1** | **Group 2** | | **Group 3** | | **Group 4** | | **Group 5** | |
|---|---|---|---|---|---|---|---|---|---|
| Rabbit ID | **liq IN CpG** | Rabbit ID | **FD IN CpG** | Rabbit ID | **SFD IN CpG** | Rabbit ID | **FD IN CpG+Chit** | Rabbit ID | **SFD IN CpG+Chit** |
| 1-2 <2 | | 2-3 | 2 | 3-1 | <2 | 4-2 | 4 | 5-4 | 16 |
| 1-3 | 16 | 2-5 | <2 | 3-6 | <2 | 4-3 | <2 | 5-5 | 4 |
| 1-7 | 128 | 2-7 | <2 | 3-7 | 16 | 4-6 | <2 | | |
| Mean | 72 | | <2 | | 5 | | <2 | | 10 |
| **GMT** | **45** | | **<2** | | **3** | | **<2** | | **8** |
| | | | | | | | | | |

| | **Group 6** | **Group 7** | | **Group 11** | | | | | |
|---|---|---|---|---|---|---|---|---|---|
| Rabbit ID | **ID CpG** | Rabbit ID | **SMN CpG** | Rabbit ID | **IM** | | | | |
| 6-1 | 2 | 7-1 | 8 | 11-3 | 8 | | | | |
| 6-3 | 8 | 7-6 | <2 | 11-5 | <2 | | | | |
| 6-9 | 8 | 7-9 | 2 | 11-6 | 16 | | | | |
| Mean | 6 | | 5 | | 12 | | | | |
| **GMT** | **5** | **3** | | | **5** | | | | |

**Table 11. Nasal IgA Titres (day 56)**

| | **Group 1** | | **Group 2** | | **Group 3** | | **Group 4** | | **Group 5** |
|---|---|---|---|---|---|---|---|---|---|
| Rabbit ID | **liq IN CpG** | Rabbit ID | **FD IN CpG** | Rabbit ID | **SFD IN CpG** | Rabbit ID | **FD IN CpG+Chit** | Rabbit ID | **SFD IN CpG+Chit** |
| 1-2 | 8 | 2-3 | <2 | 3-1 | 4 | 4-2 | 4 | 5-4 | <2 |
| 1-3 | 16 | 2-5 | 4 | 3-6 | 8 | 4-3 | <2 | 5-5 | <2 |
| 1-7 | 32 | 2-7 | 8 | 3-7 | 32 | 4-6 | 4 | | |
| Mean | 19 | | 6 | | 15 | | 3 | | <2 |
| **GMT** | **16** | | **3** | | **10** | | **3** | | <2 |
| | | | | | | | | | |

| | **Groups 6** | | **Group 7** | | **Group 11** | | | | |
|---|---|---|---|---|---|---|---|---|---|
| Rabbit ID | ID **CpG** | Rabbit ID | **SMN CpG** | Rabbit | ID **IM** | | | | |
| 6-1 | <2 | 7-1 | 16 | 11-3 | 4 | | | | |
| 6-3 | 2 | 7-6 | 2 | 11-5 | 4 | | | | |
| 6-9 | <2 | 7-9 | 16 | 11-6 | <2 | | | | |
| Mean | <2 | | 11 | | 3 | | | | |
| **GMT** | **<2** | | **8** | | **3** | | | | |

**Table 12. Vaginal IgA Titres (day 56)**

| | **Group 1** | | **Group 2** | | **Group 3** | | **Group 4** | | **Group 5** |
|---|---|---|---|---|---|---|---|---|---|
| Rabbit ID | **liq IN CpG** | Rabbit ID | **FD IN CpG** | Rabbit ID | **SFD IN CpG** | Rabbit ID | **FD IN CpG+Chit** | Rabbit ID | **SFD IN CpG+Chit** |
| 1-2 | <5 | 2-3 | 5 | 3-1 | <5 | 4-2 | <5 | 5-4 | <5 |
| 1-3 | 5 | 2-5 | <5 | 3-6 | <5 | 4-3 | <5 | 5-5 | <5 |
| 1-7 | <5 | 2-7 | <5 | 3-7 | <5 | 4-6 | <5 | | |
| Mean | <5 | | <5 | | <5 | | <5 | | <5 |
| **GMT** | **<5** | | **<5** | | **<5** | | **<5** | | **<5** |
| | | | | | | | | | |

| | **Group 6** | | **Group 7** | | **Group 11** | | | | |
|---|---|---|---|---|---|---|---|---|---|
| Rabbit ID | **ID CpG** | Rabbit ID | **SMN CpG** | Rabbit ID | **IM** | | | | |
| 6-1 | 5 | 7-1 | <5 | 11-3 | 5 | | | | |
| 6-3 | <5 | 7-6 | <5 | 11-5 | <5 | | | | |
| 6-9 | <5 | 7-9 | 10 | 11-6 | <5 | | | | |
| Mean | <5 | | <5 | | <5 | | | | |
| **GMT** | **<5** | | **<5** | | **<5** | | | | |

### Example 10: Neutralizing antibody response

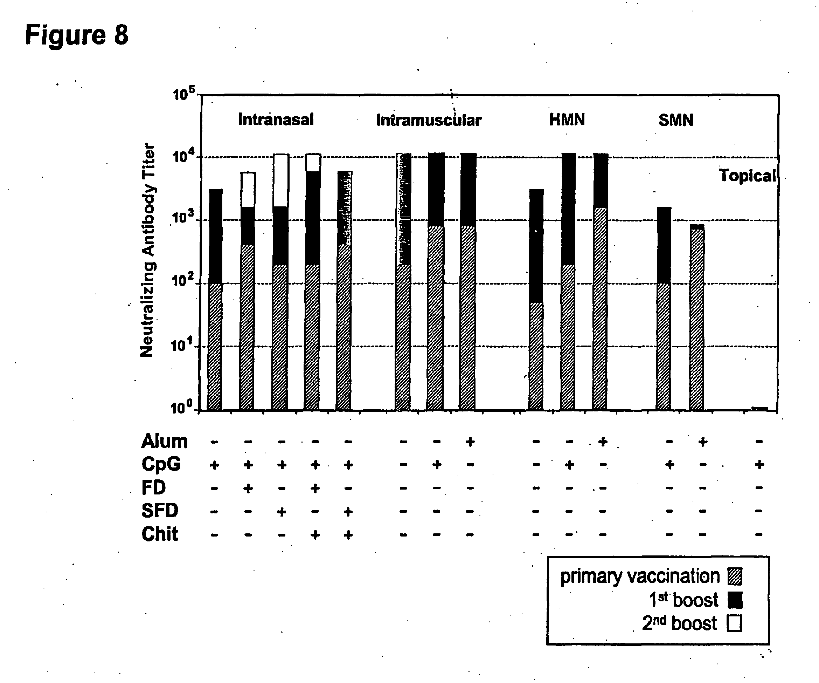

Anthrax neutralization from was measured by adding dilutions of rabbit sera from vaccinated or control animals to monocyte cell cultures incubated with lytic concentrations of PA and lethal toxin (LT) from *B. anthracis.* This assay represents an *in vitro* correlate of protection against anthrax disease. Cell viability was determined by measuring intracellular ATP. Results are presented in Figure 8. After one dose of vaccine, the highest overall Nab titers were observed in the ID-alum group; Nab levels in this group were higher than those generated in any of the IM injection groups. IN and SMN-based delivery also induced significant Nab titers after a single dose of vaccine to levels that were comparable to those induced by IM injection. After the 2^{nd} dose of vaccine, Nab titers were comparable across the IM, HMN and IN groups. A booster effect from the 3^{rd} dose of vaccine was only evident in IN groups dosed with powder formulated vaccine. SMN-delivery enabled topical delivery of anthrax vaccine, as no detectable Nab response was evident in the group treated topically in the absence of the SMN device. Thus, the vaccine composition and methods of the present invention induce Nabs at levels that are at least as strong as those induced by conventional IM injection

### Example 11 - Summary of protective immune response: Survival after aerosol challenge with anthrax spores.

Data obtained with various types of administration are summarized in Table 13.

**TABLE 13. Protection among vaccinated rabbits against aerosol Bacillus anthracis challenge**

| **Group** | **Vaccination** | | | | ***B. anthracis¹* challenge** | |
|---|---|---|---|---|---|---|
| strain | antigen³ (µg) | adjuvant³ (µg) | no. | route | LD₆₀s² *(mean* ± *SD)* | alive/total (%) |
| *naïve control NZW* | - | - | - | - | 105 ± 38 | 0/8 (0) |
| *Antigen only NZW* | | | | | | |
| | rPA (50) | none | 3 | intramuscular | 107± 49 | 6/6 (100) |
| | rPA (50) | none | 3 | HMN | 91± 63 | 6/6 (100) |
| *Antigen*/*adjuvant NZW* | | | | | | |
| | rPA (50) | alum (360) | 3 | Intramuscular | 107± 58 | 5/6 (83) |
| | rPA (50) | alum (360) | 3 | HMN | 111 ± 34 | 6/6 (100) |
| | rPA (50) | alum (360) | 3 | SMN | 11.1 ± 34 | 2/6 (33) |
| | rPA (50) | CpG (50) | 3 | topical | 80 ± 25 | 1/6 (17) |
| | rPA (50) | CpG (50) | 3 | SMN | 81 ± 47 | 3/6 (50) |
| | rPA (50) | CpG (50) | 3 | HMN | 101 ± 50 | 5/6 (83) |
| | rPA (50) | CpG (50) | 3 | intramuscular | 101 ± 70 | 5/6 (83) |
| *Antigen*/***adjuvant** NZW* | | | | | | |
| | rPA (50) | CpG (50) | 3 | intranasal⁴ | 96 ± 44 | 4/6 (67) |
| | rPA (50) | CpG (50) | 3 | Intranasal; SFD⁵ | 133 ± 35 | 5/8 (83) |
| | rPA (50) | CpG (50) | 3 | lntranasal; FD⁶ | 106 ± 54 | 8/6 (100) |
| | rPA (50) | CpG/chit. (50) | 3 | Intranasal;SFD⁵ | 113 ± 29 | 6/6 (100) |
| | rPA (50) | CpG/chit. (50) | 3 | Intranasal;FD⁶ | 101 ± 51 | 6/6 (100) |

| | | | | | | |
|---|---|---|---|---|---|---|
| ¹ Ames strain, provided by Dr. Bruce Ivins, Division of Bacteriology, USAMRIID. ² Overall number of aerosol LD₅₀s given calculated at 103 ± 44 (mean ± S.D.). ³ Amount in parenthesis is per vaccination. ⁴ Liquid antigen/adjuvant formulation. ⁵ Spray freeze-dried powder antigen/adjuvant formulation. ⁶ Freeze-dried powder antigen/adjuvant formulation. | | | | | | |

The current standard for anthrax vaccination consists of IM injection of vaccine containing alum as adjuvant. In this study, this standard provided partial protection (83%) against lethal challenge (Table 13). In contrast, HMN-based ID delivery of rPA plus alum or rPA without adjuvant provided complete protection (100%). In general, choice of adjuvant for IM or ID groups did not seem to play a major role in determining protective efficacy at the dose of rPA employed in this study. IN delivery of a liquid formulation provided 67% protection, while IN delivery of the powder formulation of the present invention provided complete protection (100%). SMN-based topical delivery provided up to 50% protection as compared to only 17% by topical administration in the absence of a device. Overall, the results of the lethal challenge study demonstrate that by targeting the skin and nasal mucosa via the methods and formulations of the present invention, complete protection against anthrax can be achieved. This is the first time that protection has been demonstrated through targeting of the very tissues through which anthrax disease is acquired in natural and weaponized forms of the pathogen (i.e, skin and respiratory systems).

### Example 12 - Dose-sparing benefits

The results of the mouse and rabbit studies presented above show that ID delivery via HMN provides stronger immune responses than IM injection after a single dose of vaccine. Thus, ID delivery via HMN may enable a reduction in the number of required doses of anthrax vaccine to elicit protective effect. Also, based on these results, it is anticipated that ID delivery via HMN may provide dose sparing benefits; i.e., may enable one to use a reduced amount of rPA per dosage to achieve the same or greater level of immune response and protection as that achieved using the full dose of vaccine via the IM route.

## Claims

1. An immunogenic anthrax composition comprising dried solid particles having a volume mean diameter of between 35 µm and 300 µm, wherein at least 50% of said dried particles have a volume diameter within 80% of the mean, and said dried particles have a mean aerodynamic diameter of between 8 µm and 140 µm.

2. The immunogenic anthrax composition of claim 1, wherein the composition is made by a method comprising the steps of:
(i) atomizing a liquid formulation of an immunogenic anthrax composition to produce an atomized formulation,
(ii) freezing said atomized formulation to form solid particles, and
(iii) drying said solid particles to obtain dried particles.

3. The composition of claim 1 or 2 that is an anthrax vaccine.

4. The composition of claim 3, wherein the anthrax vaccine:
(i) is an Anthrax Vaccine Absorbed (AVA) vaccine; or
(ii) is a recombinant protective antigen (rPA) vaccine; or
(iii) comprises an adjuvant; or
(iv) does not contain an adjuvant; or
(v) comprises dried particles that have a volume mean diameter of between 50 µm and 300 µm; or
(vi) is reconstituted to form a liquid formulation consisting essentially of said anthrax vaccine and water.

5. The composition of claim 3, wherein the anthrax vaccine comprises an immunogenic agent and at least one pharmaceutically acceptable carrier.

6. The composition of Claim 5, wherein the anthrax vaccine:
(i) is an AVA vaccine ; or
(ii) is an rPA vaccine.

7. The composition of claim 3, wherein the anthrax vaccine is made by a method comprising
(i) atomizing a liquid formulation of an anthrax vaccine composition to produce an atomized formulation which comprises droplets having an average mean diameter of between 35 µm and 300 µm,
(ii) freezing said atomized formulation to form solid particles, and
(iii) drying said solid particles to obtain dried particles.

8. A method of preparing an anthrax vaccine, comprising
(i) atomizing a liquid formulation of an anthrax vaccine composition to produce an atomized formulation,
(ii) freezing said atomized formulation to form solid particles, and
(iii) drying said solid particles to obtain dried particles, wherein said dried particles have an average mean diameter of between 35 µm and 300 µm, at least 50% of said dried particles have a volume diameter within 80% of the mean, and said dried particles have a mean aerodynamic diameter of between 8 µm and 140 µm, preferably, said dried particles have a volume mean diameter of between 50 µm and 300 µm.

9. The method of claim 8, wherein said liquid formulation of said anthrax vaccine composition consists essentially of said anthrax vaccine and water.

10. Use of the immunogenic anthrax composition of claim 1 for preparing an anthrax vaccine for eliciting an immune response in a patient.

11. The use of claim 10, wherein:
(i) the anthrax vaccine is an Anthrax Vaccine Absorbed (AVA) vaccine; or
(ii) the anthrax vaccine is a recombinant protective antigen (rPA) vaccine; or
(iii) the anthrax vaccine comprises an adjuvant; or
(iv) the anthrax vaccine does not contain an adjuvant; or
(v) the anthrax vaccine is suitable for intranasal delivery; or
(vi) the anthrax vaccine is suitable for delivery to a mucosal membrane; or
(vii) the anthrax vaccine is suitable for reducing the amount of an anthrax vaccine that is required to produce an efficacious result by intranasal administration .

12. The use of claim 10, wherein said vaccine is suitable for intranasal delivery.

13. The use of claim 10 or 11, wherein the vaccine is suitable for vaccinating a patient against anthrax.

14. The use of claim 13, wherein said vaccine is suitable
(i) for intranasal delivery; or
(ii) to be administered as part of a prime-boost regimen; or
(iii) for intradermal delivery at a depth between 0.025 mm and 2.5 mm in the skin of the patient; preferably the medicament is suitable for delivery through at least one hollow needle having an outlet with an exposed height between 0 and 1 mm, said outlet being insertable into the skin to a depth of between 0.5 mm and 2 mm.

15. The use of claim 10, wherein the vaccine induces an immune response in a mammal that is equal to or greater than the response after delivery of the same amount of medicament by subcutaneous or intramuscular injection.

16. The use of claim 15, wherein the needle is a microneedle between 0.23 and 0.025 mm (31 and 50 gauge), preferably the microneedle is in an array of microneedles.

17. The use of claim 11 for the expression of protective immunity by delivery of said immunogenic anthrax vaccine into an intradermal space within the skin of a human subject through a small gauge needle inserted into the intradermal space, the needle having an outlet having an exposed height with an exposed height between 0 and 1 mm and inserted at a depth between 0.025 mm and 2.5 mm in the skin, whereby the immunogenicity of the anthrax vaccine is improved.

18. The use of claim 11 for the expression of protective immunity by delivery of said immunogenic anthrax vaccine into the skin of a human subject by abrading the skin and delivering the anthrax vaccine into the abraded skin wherein the skin is
(i) abraded prior to delivery of the anthrax vaccine; or
(ii) abraded simultaneously with the delivery of the anthrax vaccine, whereby the immunogenicity of the anthrax vaccine is improved.

19. The use of Claim 17 or 18, wherein:
(i) the anthrax vaccine is an Anthrax Vaccine Absorbed (AVA) vaccine; or
(ii) the anthrax vaccine is a recombinant protective antigen (rPA) vaccine; or
(iii) the anthrax vaccine comprises an adjuvant; or
(iv) the anthrax vaccine does not contain an adjuvant.

20. The use of Claim 18, wherein the skin is abraded at least twice.

21. The use of Claim 18, wherein the skin is abraded in alternating directions.

22. The use of Claim 18, wherein the immunogenic anthrax vaccine is applied to the skin in liquid form.

23. The use of Claim 18, wherein the immunogenic anthrax vaccine is applied to the skin from an abrading device.

24. The use of Claim 23, wherein the immunogenic anthrax vaccine is a powder, gel or film on an abrading surface of an abrading device.

25. The use of Claim 24, wherein a reconstituting liquid is separately applied to the skin prior to abrasion.

26. The use of Claim 24, wherein a reconstituting liquid is applied to the skin simultaneously with abrasion.

27. A kit comprising an effective amount of an anthrax vaccine of claim 3.

28. The kit of claim 27 that further comprises means to administer the vaccine intranasally.

29. The kit of claim 27 comprising a microabrader comprising an abrading surface, and an effective amount of an anthrax vaccine.

30. The kit of Claim 29, wherein:
(i) the anthrax vaccine is coated on the surface of the microabrader; or
(ii) the anthrax vaccine is contained in a reservoir integrated with the microabrader.

31. The kit of Claim 27, wherein the anthrax vaccine is separately packaged within the kit.

32. The kit of claim 27 comprising a delivery device suitable for intradermal delivery of a substance to a depth between 0.025 and 2.5 mm of the skin of a patient, and an effective dosage of an anthrax vaccine.

33. The kit of claim 32 wherein said delivery device comprises at least one hollow microneedle designed for intradermal delivery of a substance to a depth between 0.025 and 2.5 mm of the skin of a patient, and the delivery device comprises, or is adapted to receive a reservoir that contains an anthrax vaccine such that the microneedle is in communication therewith.

34. The kit of claim 33 wherein
(i) the dosage is contained in a reservoir that is an integral part of, or is capable of being functionally attached to, the delivery device; or
(ii) the hollow microneedle is between 0.23 and 0.025 mm (31 and 50 gauge); or
(iii) the device comprises an array of microneedles.

## Patentansprüche

1. Immunogene Milzbrandzusammensetzung, die getrocknete feste Teilchen mit einem Volumenmittel des Durchmessers zwischen 35 µm und 300 µm umfasst, wobei wenigstens 50% der getrockneten Teilchen einen Volumendurchmesser innerhalb von 80% des Mittelwerts haben und die getrockneten Teilchen einen mittleren aerodynamischen Durchmesser zwischen 8 µm und 140 µm haben.

2. Immunogene Milzbrandzusammensetzung gemäß Anspruch 1, wobei die Zusammensetzung nach einem Verfahren hergestellt wird, das die folgenden Schritte umfasst:
(i) Zerstäuben einer flüssigen Zubereitung einer immunogenen Milzbrandzusammensetzung unter Bildung einer zerstäubten Zubereitung;
(ii) Gefrierenlassen der zerstäubten Zubereitung unter Bildung von festen Teilchen; und
(iii) Trocknen der festen Teilchen, wobei man getrocknete Teilchen erhält.

3. Zusammensetzung gemäß Anspruch 1 oder 2, bei der es sich um einen Milzbrand-Impfstoff handelt.

4. Zusammensetzung gemäß Anspruch 3, wobei der Milzbrand-Impfstoff:
(i) ein AVA-Impfstoff (anthrax vaccine absorbed) ist; oder
(ii) ein Impfstoff mit einem rekombinanten Schutzantigen (rPA) ist; oder
(iii) ein Adjuvans umfasst; oder
(iv) kein Adjuvans enthält; oder
(v) getrocknete Teilchen umfasst, die ein Volumenmittel des Durchmessers zwischen 50 µm und 300 µm aufweisen; oder
(vi) unter Bildung einer flüssigen Zusammensetzung, die im Wesentlichen aus dem Milzbrand-Impfstoff und Wasser besteht, rekonstituiert wird.

5. Zusammensetzung gemäß Anspruch 3, wobei der Milzbrand-Impfstoff ein immunogenes Agens und wenigstens einen pharmazeutisch annehmbaren Träger umfasst.

6. Zusammensetzung gemäß Anspruch 5, wobei der Milzbrand-Impfstoff:
(i) ein AVA-Impfstoff ist; oder
(ii) ein rPA-Impfstoff ist.

7. Zusammensetzung gemäß Anspruch 3, wobei der Milzbrand-Impfstoff durch ein Verfahren hergestellt ist, das Folgendes umfasst:
(i) Zerstäuben einer flüssigen Zubereitung einer Milzbrand-Impfstoffzusammensetzung unter Bildung einer zerstäubten Zubereitung, die Tröpfchen mit einem durchschnittlichen mittleren Durchmesser zwischen 35 µm und 300 µm umfasst;
(ii) Gefrierenlassen der zerstäubten Zubereitung unter Bildung von festen Teilchen; und
(iii) Trocknen der festen Teilchen, wobei man getrocknete Teilchen erhält.

8. Verfahren zur Herstellung eines Milzbrand-Impfstoffs, umfassend:
(i) Zerstäuben einer flüssigen Zubereitung einer Milzbrand-Impfstoffzusammensetzung unter Bildung einer zerstäubten Zubereitung;
(ii) Gefrierenlassen der zerstäubten Zubereitung unter Bildung von festen Teilchen; und
(iii) Trocknen der festen Teilchen, wobei man getrocknete Teilchen erhält, wobei die getrockneten Teilchen einen durchschnittlichen mittleren Durchmesser zwischen 35 µm und 300 µm aufweisen, wenigstens 50% der getrockneten Teilchen einen Volumendurchmesser innerhalb von 80% des Mittelwerts haben und die getrockneten Teilchen einen mittleren aerodynamischen Durchmesser zwischen 8 µm und 140 µm haben, wobei die getrockneten Teilchen vorzugsweise ein Volumenmittel des Durchmessers zwischen 50 µm und 300 µm haben.

9. Verfahren gemäß Anspruch 8, wobei die flüssige Zubereitung der Milzbrand-Impfstoffzusammensetzung im Wesentlichen aus dem Milzbrand-Impfstoff und Wasser besteht.

10. Verwendung der immunogenen Milzbrandzusammensetzung gemäß Anspruch 1 zur Herstellung eines Milzbrand-Impfstoffs zum Hervorrufen einer Immunantwort bei einem Patienten.

11. Verwendung gemäß Anspruch 10, wobei:
(i) der Milzbrand-Impfstoff ein AVA-Impfstoff (anthrax vaccine absorbed) ist; oder
(ii) der Milzbrand-Impfstoff ein Impfstoff mit einem rekombinanten Schutzantigen (rPA) ist; oder
(iii) der Milzbrand-Impfstoff ein Adjuvans umfasst; oder
(iv) der Milzbrand-Impfstoff kein Adjuvans enthält; oder
(v) der Milzbrand-Impfstoff für die intranasale Verabreichung geeignet ist; oder
(vi) der Milzbrand-Impfstoff für die Verabreichung über eine Schleimhaut geeignet ist; oder
(vii) der Milzbrand-Impfstoff geeignet ist, die Menge eines Milzbrand-Impfstoffs, die erforderlich ist, um durch intranasale Verabreichung ein wirksames Ergebnis zu erhalten, zu reduzieren.

12. Verwendung gemäß Anspruch 10, wobei der Impfstoff für die intranasale Verabreichung geeignet ist.

13. Verwendung gemäß Anspruch 10 oder 11, wobei der Impfstoff zum Impfen eines Patienten gegen Milzbrand geeignet ist.

14. Verwendung gemäß Anspruch 13, wobei der Impfstoff geeignet ist
(i) für die intranasale Verabreichung; oder
(ii) zur Verabreichung als Teil eines Prime/Boost-Impfschemas; oder
(iii) für die intradermale Verabreichung in einer Tiefe zwischen 0,025 mm und 2,5 mm in der Haut des Patienten, wobei das Medikament vorzugsweise geeignet ist zur Verabreichung durch wenigstens eine Hohlnadel mit einem Auslass mit einer exponierten Höhe zwischen 0 und 1 mm, wobei der Auslass bis zu einer Tiefe zwischen 0,5 mm und 2 mm in die Haut eingeführt werden kann.

15. Verwendung gemäß Anspruch 10, wobei der Impfstoff in einem Säuger eine Immunantwort induziert, die gleich oder größer ist als die Antwort nach Verabreichung derselben Menge an Medikament durch subkutane oder intramuskuläre Injektion.

16. Verwendung gemäß Anspruch 15, wobei die Nadel eine Mikronadel mit einem Durchmesser zwischen 0,23 und 0,025 mm (Nr. 31 bzw. 50) ist und die Mikronadel vorzugsweise in einem Feld von Mikronadeln vorliegt.

17. Verwendung gemäß Anspruch 11 zur Expression von schützender Immunität durch Verabreichung des immunogenen Milzbrand-Impfstoffs in einen intradermalen Raum innerhalb der Haut eines humanen Patienten durch eine Nadel mit kleinem Durchmesser, die in den intradermalen Raum eingeführt wird, wobei die Nadel einen Auslass mit einer exponierten Höhe zwischen 0 und 1 mm aufweist und in einer Tiefe zwischen 0,025 mm und 2,5 mm in die Haut eingeführt wird, wodurch die Immunogenität des Milzbrand-Impfstoffs verbessert wird.

18. Verwendung gemäß Anspruch 11 zur Expression von schützender Immunität durch Verabreichung des immunogenen Milzbrand-Impfstoffs in die Haut eines humanen Patienten durch Abscheuern der Haut und Verabreichen des Milzbrand-Impfstoffs in die abgescheuerte Haut, wobei die Haut
(i) vor der Verabreichung des Milzbrand-Impfstoffs abgescheuert wird; oder
(ii) gleichzeitig mit der Verabreichung des Milzbrand-Impfstoffs abgescheuert wird;
wodurch die Immunogenität des Milzbrand-Impfstoffs verbessert wird.

19. Verwendung gemäß Anspruch 17 oder 18, wobei:
(i) der Milzbrand-Impfstoff ein AVA-Impfstoff (anthrax vaccine absorbed) ist; oder
(ii) der Milzbrand-Impfstoff ein Impfstoff mit einem rekombinanten Schutzantigen (rPA) ist; oder
(iii) der Milzbrand-Impfstoff ein Adjuvans umfasst; oder
(iv) der Milzbrand-Impfstoff kein Adjuvans enthält.

20. Verwendung gemäß Anspruch 18, wobei die Haut wenigstens zweimal abgescheuert wird.

21. Verwendung gemäß Anspruch 18, wobei die Haut in abwechselnden Richtungen abgescheuert wird.

22. Verwendung gemäß Anspruch 18, wobei der immunogene Milzbrand-Impfstoff in flüssiger Form auf die Haut aufgetragen wird.

23. Verwendung gemäß Anspruch 18, wobei der immunogene Milzbrand-Impfstoff von einer Scheuervorrichtung aus auf die Haut aufgetragen wird.

24. Verwendung gemäß Anspruch 23, wobei der immunogene Milzbrand-Impfstoff ein Pulver, Gel oder Film auf einer Scheuerfläche einer Scheuervorrichtung ist.

25. Verwendung gemäß Anspruch 24, wobei vor dem Abscheuern eine rekonstituierende Flüssigkeit separat auf die Haut aufgetragen wird.

26. Verwendung gemäß Anspruch 24, wobei gleichzeitig mit dem Abscheuern eine rekonstituierende Flüssigkeit auf die Haut aufgetragen wird.

27. Kit, der eine wirksame Menge eines Milzbrand-Impfstoffs gemäß Anspruch 3 umfasst.

28. Kit gemäß Anspruch 27, der weiterhin Mittel zur intranasalen Verabreichung des Impfstoffs umfasst.

29. Kit gemäß Anspruch 27, der eine Mikroscheuervorrichtung, die eine Scheuerfläche umfasst, und eine wirksame Menge eines Milzbrand-Impfstoffs umfasst.

30. Kit gemäß Anspruch 29, wobei:
(i) der Milzbrand-Impfstoff auf die Oberfläche der Mikroscheuervorrichtung aufgetragen ist; oder
(ii) der Milzbrand-Impfstoff in einem einstückig mit der Mikroscheuervorrichtung ausgebildeten Reservoir enthalten ist.

31. Kit gemäß Anspruch 27, wobei der Milzbrand-Impfstoff innerhalb des Kits separat verpackt ist.

32. Kit gemäß Anspruch 27, der eine Verabreichungsvorrichtung, die für die intradermale Verabreichung einer Substanz bis zu einer Tiefe zwischen 0,025 mm und 2,5 mm der Haut eines Patienten geeignet ist, und eine wirksame Dosis eines Milzbrand-Impfstoffs umfasst.

33. Kit gemäß Anspruch 32, wobei die Verabreichungsvorrichtung wenigstens eine hohle Mikronadel umfasst, die für die intradermale Verabreichung einer Substanz bis zu einer Tiefe zwischen 0,025 mm und 2,5 mm der Haut eines Patienten vorgesehen ist, und die Verabreichungsvorrichtung ein Reservoir, das einen Milzbrand-Impfstoff enthält, in einer solchen Weise umfasst oder aufnehmen kann, dass die Mikronadel damit kommuniziert.

34. Kit gemäß Anspruch 33, wobei
(i) die Dosis in einem Reservoir enthalten ist, das ein integraler Bestandteil der Verabreichungsvorrichtung ist oder funktionell mit dieser verbunden werden kann; oder
(ii) die hohle Mikronadel einen Durchmesser zwischen 0,23 und 0,025 mm (Nr. 31 bzw. 50) hat; oder
(iii) die Vorrichtung ein Feld von Mikronadeln umfasst.

## Revendications

1. Composition d'anthrax immunogène comprenant des particules solides séchées ayant un diamètre volumétrique moyen compris entre 35 µm et 300 µm, dans laquelle au moins 50% desdites particules séchées ont un diamètre volumétrique dans les 80% de la moyenne, et lesdites particules séchées ont un diamètre aérodynamique moyen compris entre 8 µm et 140 µm.

2. Composition d'anthrax immunogène selon la revendication 1, ladite composition étant fabriquée à l'aide d'un procédé comprenant les étapes consistant à:
(i) atomiser une formulation liquide d'une composition d'anthrax immunogène pour produire une formulation atomisée,
(ii) congeler ladite formulation atomisée pour former des particules solides, et
(iii) sécher lesdites particules solides pour obtenir des particules séchées.

3. Composition selon la revendication 1 ou 2, qui est un vaccin contre le charbon.

4. Composition selon la revendication 3, où le vaccin contre le charbon:
(i) est un vaccin absorbé sur du vaccin contre le charbon (Anthrax Vaccine Absorbed, AVA); ou '
(ii) est un vaccin à base d'antigène protecteur recombinant (APr); ou
(iii) comprend un adjuvant; ou
(iv) ne contient pas d'adjuvant; ou
(v) comprend des particules séchées qui ont un diamètre volumétrique moyen compris entre 50 µm et 300 µm; ou
(iv) est reconstitué pour former une formulation liquide constituée essentiellement dudit vaccin contre le charbon et d'eau.

5. Composition selon la revendication 3, où le vaccin contre le charbon comprend un agent immunogène et au moins un véhicule pharmaceutiquement acceptable.

6. Composition selon la revendication 5, où le vaccin contre le charbon:
(i) est un vaccin AVA; ou
(ii) est un vaccin à base d'APr.

7. Composition selon la revendication 3, où le vaccin contre le charbon est fabriqué à l'aide d'un procédé comprenant:
(i) l'atomisation d'une formulation liquide d'une composition de vaccin contre le charbon pour produire une formulation atomisée qui comprend des gouttelettes ayant un écart moyen du diamètre compris entre 35 µm et 300 µm,
(ii) la congélation de ladite formulation atomisée pour former des particules solides, et
(iii) le séchage desdites particules solides pour obtenir des particules séchées.

8. Procédé de préparation d'un vaccin contre le charbon, comprenant:
(i) l'atomisation d'une formulation liquide d'une composition de vaccin contre le charbon pour produire une formulation atomisée,
(ii) la congélation de ladite formulation atomisée pour former des particules solides, et
(iii) le séchage desdites particules solides pour obtenir des particules séchées, dans lequel lesdites particules séchées ont un écart moyen du diamètre compris entre 35 µm et 300 µm, au moins 50% desdites particules séchées ont un diamètre volumétrique dans les 80% de la moyenne, et lesdites particules séchées ont un diamètre aérodynamique moyen compris entre 8 µm et 140 µm, de préférence, lesdites particules séchées ont un diamètre volumétrique moyen compris entre 50 µm et 300 µm.

9. Procédé selon la revendication 8, dans lequel ladite formulation liquide de ladite composition de vaccin contre le charbon est constituée essentiellement dudit vaccin contre le charbon et d'eau.

10. Utilisation de la composition d'anthrax immunogène selon la revendication 1 pour préparer un vaccin contre le charbon permettant de déclencher une réponse immunitaire chez un patient.

11. Utilisation selon la revendication 10, où:
(i) le vaccin contre le charbon est un vaccin absorbé sur du vaccin contre le charbon (Anthrax Vaccine Absorbed, AVA); ou
(ii) le vaccin contre le charbon est un vaccin à base d'antigène protecteur recombinant (APr); ou
(iii) le vaccin contre le charbon comprend un adjuvant; ou
(vi) le vaccin contre le charbon ne contient pas d'adjuvant; ou
(v) le vaccin contre le charbon convient pour une délivrance intranasale; ou
(iv) le vaccin contre le charbon convient pour une délivrance à une membrane muqueuse; ou
(vii) le vaccin contre le charbon convient pour une réduction de la quantité de vaccin contre le charbon qui est nécessaire pour produire un résultat efficace par administration intranasale.

12. Utilisation selon la revendication 10, où ledit vaccin convient pour une délivrance intranasale.

13. Utilisation selon la revendication 10 ou 11, où le vaccin convient pour vacciner un patient contre le charbon.

14. Utilisation selon la revendication 13, où ledit vaccin convient
(i) pour une délivrance intranasale; ou
(ii) pour être administré dans le cadre d'un régime de premier rappel; ou
(iii) pour la délivrance intradermique à une profondeur comprise entre 0,025 mm et 2,5 mm dans la peau du patient; de préférence, le médicament convient pour une délivrance à travers au moins une aiguille creuse dont l'orifice de sortie a une hauteur exposée comprise entre 0 et 1 mm, ledit orifice de sortie étant insérable dans la peau à une profondeur comprise entre 0,5 mm et 2 mm.

15. Utilisation selon la revendication 10, où le vaccin induit chez un mammifère une réponse immunitaire qui est supérieure ou égale à la réponse après la délivrance de la même quantité de médicament par injection sous-cutanée ou intramusculaire.

16. Utilisation selon la revendication 15, où l'aiguille est une microaiguille comprise entre 0,23 et 0,025 mm (calibre 31 et 50), de préférence, la microaiguille est dans une barrette de microaiguilles.

17. Utilisation selon la revendication 11 pour l'expression d'une immunité protectrice par délivrance dudit vaccin immunogène contre le charbon dans un espace intradermique au sein de la peau d'un sujet humain par l'intermédiaire d'une aiguille de faible calibre insérée dans l'espace intradermique, l'aiguille ayant un orifice de sortie ayant une hauteur exposée, la hauteur exposée étant comprise entre 0 et 1 mm, et insérée à une profondeur comprise entre 0,025 mm et 2,5 mm dans la peau, si bien que l'immunogénicité du vaccin contre le charbon est améliorée.

18. Utilisation selon la revendication 11 pour l'expression d'une immunité protectrice par délivrance dudit vaccin immunogène contre le charbon dans la peau d'un sujet humain en abrasant la peau et en délivrant le vaccin contre le charbon dans la peau abrasée, où la peau est
(i) abrasée avant la délivrance du vaccin contre le charbon; ou
(ii) abrasée simultanément avec la délivrance du vaccin contre le charbon, si bien que l'immunogénicité du vaccin contre le charbon est améliorée.

19. Utilisation selon la revendication 17 ou 18, où:
(i) le vaccin contre le charbon est un vaccin absorbé sur du vaccin contre le charbon (Anthrax Vaccine Absorbed, AVA); ou
(ii) le vaccin contre le charbon est un vaccin à base d'antigène protecteur recombinant (APr); ou
(iii) le vaccin contre le charbon comprend un adjuvant; ou
(iv) le vaccin contre le charbon ne contient pas d'adjuvant.

20. Utilisation selon la revendication 18, où la peau est abrasée au moins deux fois.

21. Utilisation selon la revendication 18, où la peau est abrasée alternativement dans un sens et dans l'autre.

22. Utilisation selon la revendication 18, où le vaccin immunogène contre le charbon est appliqué sur la peau sous forme liquide.

23. Utilisation selon la revendication 18, où le vaccin immunogène contre le charbon est appliqué sur la peau à partir d'un dispositif d'abrasion.

24. Utilisation selon la revendication 23, où le vaccin immunogène contre le charbon est une poudre, un gel ou un film sur une surface abrasive d'un dispositif d'abrasion.

25. Utilisation selon la revendication 24, où un liquide reconstituant est appliqué séparément sur la peau avant l'abrasion.

26. Utilisation selon la revendication 24, où un liquide reconstituant est appliqué sur la peau simultanément avec l'abrasion.

27. Trousse comprenant une quantité efficace d'un vaccin contre le charbon selon la revendication 3.

28. Trousse selon la revendication 27, qui comprend en outre un moyen pour administrer le vaccin par voie intranasale.

29. Trousse selon la revendication 27 comprenant un microabraseur comportant une surface abrasive, et une quantité efficace d'un vaccin contre le charbon.

30. Trousse selon la revendication 29, dans laquelle:
(i) le vaccin contre le charbon est appliqué à la surface du microabraseur; ou
(ii) le vaccin contre le charbon est contenu dans un réservoir intégré au microabraseur.

31. Trousse selon la revendication 27, dans laquelle le vaccin contre le charbon est conditionné séparément à l'intérieur de la trousse.

32. Trousse selon la revendication 27 comprenant un dispositif de délivrance approprié pour la délivrance intradermique d'une substance à une profondeur comprise entre 0,025 et 2,5 mm de la peau d'un patient, et une dose efficace d'un vaccin contre le charbon.

33. Trousse selon la revendication 32, dans laquelle ledit dispositif de délivrance comprend au moins une microaiguille creuse conçue pour la délivrance intradermique d'une substance à une profondeur comprise entre 0,025 et 2,5 mm de la peau d'un patient, et le dispositif de délivrance comprend, ou est adapté pour recevoir, un réservoir qui contient un vaccin contre le charbon, de sorte que la microaiguille communique avec celui-ci.

34. Trousse selon la revendication 33, dans laquelle
(i) la dose est contenue dans un réservoir qui fait partie intégrante du, ou qui peut se fixer de manière fonctionnelle au, dispositif de délivrance; ou
(ii) la microaiguille creuse est comprise entre 0,23 et 0,025 mm (calibre 31 1 et 50); ou
(iii) le dispositif comprend une barrette de microaiguilles.
